(19)

Europäisches
Patentamt

European
Patent Office

Office européen
des brevets

(11) **EP 3 156 800 A1**

(12) **EUROPEAN PATENT APPLICATION**

(43) Date of publication:
**19.04.2017 Bulletin 2017/16**

(51) Int Cl.:
**G01N 33/574** *(2006.01)*

(21) Application number: **16184951.8**

(22) Date of filing: **18.07.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.07.2010 EP 10170008**

(62) Document number(s) of the earlier application(s) in accordance with Art. 76 EPC:
**11746191.3 / 2 596 363**

(71) Applicant: **F. Hoffmann-La Roche AG**
**4070 Basel (CH)**

(72) Inventors:
• **DE HAAS, Sanne Lysbet**
**4056 Basel (CH)**
• **DELMAR, Paul**
**4057 Basel (CH)**

• **FOERNZLER, Dorothee**
**5600 Lenzburg (CH)**
• **KLAUSE, Ursula**
**82380 Peissenberg (DE)**
• **SCHERER, Stefan**
**South San Francisco, CA 94080 (US)**

(74) Representative: **Vossius & Partner**
**Patentanwälte Rechtsanwälte mbB**
**Siebertstrasse 3**
**81675 München (DE)**

Remarks:
•This application was filed on 19-08-2016 as a divisional application to the application mentioned under INID code 62.
•Claims filed after the date of filing of the application / after the date of receipt of the divisional application (Rule 68(4) EPC).

(54) **BLOOD PLASMA BIOMARKERS FOR BEVACIZUMAB COMBINATION THERAPIES FOR TREATMENT OF BREAST CANCER**

(57) The present invention provides methods for improving the treatment effect of a chemotherapy regimen of a patient suffering from breast cancer, in particular locally advanced, recurrent or metastatic HER-2 negative breast cancer, by adding bevacizumab (Avastin®) to a chemotherapy regimen by determining the expression level, in particular the blood plasma expression level, of one or more of VEGFA, VEGFR2 and PLGF relative to control levels of patients diagnosed with breast cancer, in particular locally advanced, recurrent or metastatic HER-2 negative breast cancer. In particular, the present invention provides methods of improving the treatment effect, wherein the treatment effect is the progression-free survival of the patient. The present invention further provides for methods for assessing the sensitivity or responsiveness of a patient to bevacizumab (Avastin®) in combination with a chemotherapy regimen, by determining the expression level, in particular the blood plasma expression level, of one or more of VEGFA, VEGFR2 and PLGF relative to control levels in patients diagnosed with breast cancer, in particular locally advanced, recurrent or metastatic HER-2 negative breast cancer.

EP 3 156 800 A1

SEQUENCE LISTING

<110>  F. Hoffmann-La Roche AG et al.

<120>  Blood plasma biomarkers for Bevacizumab combination therapies for treatment of breast cancer

<130>  S2247 EP/1 S3

<140>
<141>  2011-07-18

<150>  EP 10 17 0008.6
<151>  2010-07-19

<160>  3

<170>  PatentIn version 3.3

<210>  1
<211>  232
<212>  PRT
<213>  Homo sapiens

<400>  1

Met Asn Phe Leu Leu Ser Trp Val His Trp Ser Leu Ala Leu Leu Leu
1               5                   10                  15

Tyr Leu His His Ala Lys Trp Ser Gln Ala Ala Pro Met Ala Glu Gly
            20                  25                  30

Gly Gly Gln Asn His His Glu Val Val Lys Phe Met Asp Val Tyr Gln
            35                  40                  45

Arg Ser Tyr Cys His Pro Ile Glu Thr Leu Val Asp Ile Phe Gln Glu
        50              55                  60

Tyr Pro Asp Glu Ile Glu Tyr Ile Phe Lys Pro Ser Cys Val Pro Leu
65                  70                  75                  80

Met Arg Cys Gly Gly Cys Cys Asn Asp Glu Gly Leu Glu Cys Val Pro
                85                  90                  95

Thr Glu Glu Ser Asn Ile Thr Met Gln Ile Met Arg Ile Lys Pro His
                100                 105                 110

Gln Gly Gln His Ile Gly Glu Met Ser Phe Leu Gln His Asn Lys Cys
            115                 120                 125

Glu Cys Arg Pro Lys Lys Asp Arg Ala Arg Gln Glu Lys Lys Ser Val
        130                 135                 140

Arg Gly Lys Gly Lys Gly Gln Lys Arg Lys Arg Lys Lys Ser Arg Tyr

| | | | | 145 | | | | | 150 | | | | | 155 | | | | | 160 |

Lys Ser Trp Ser Val Tyr Val Gly Ala Arg Cys Cys Leu Met Pro Trp
               165             170             175

Ser Leu Pro Gly Pro His Pro Cys Gly Pro Cys Ser Glu Arg Arg Lys
           180             185            190

His Leu Phe Val Gln Asp Pro Gln Thr Cys Lys Cys Ser Cys Lys Asn
           195             200            205

Thr Asp Ser Arg Cys Lys Ala Arg Gln Leu Glu Leu Asn Glu Arg Thr
           210             215            220

Cys Arg Cys Asp Lys Pro Arg Arg
225             230

<210> 2
<211> 1356
<212> PRT
<213> Homo sapiens

<400> 2

Met Gln Ser Lys Val Leu Leu Ala Val Ala Leu Trp Leu Cys Val Glu
1          5             10            15

Thr Arg Ala Ala Ser Val Gly Leu Pro Ser Val Ser Leu Asp Leu Pro
          20             25            30

Arg Leu Ser Ile Gln Lys Asp Ile Leu Thr Ile Lys Ala Asn Thr Thr
          35             40            45

Leu Gln Ile Thr Cys Arg Gly Gln Arg Asp Leu Asp Trp Leu Trp Pro
          50             55            60

Asn Asn Gln Ser Gly Ser Glu Gln Arg Val Glu Val Thr Glu Cys Ser
65           70             75            80

Asp Gly Leu Phe Cys Lys Thr Leu Thr Ile Pro Lys Val Ile Gly Asn
          85             90           95

Asp Thr Gly Ala Tyr Lys Cys Phe Tyr Arg Glu Thr Asp Leu Ala Ser
          100           105          110

Val Ile Tyr Val Tyr Val Gln Asp Tyr Arg Ser Pro Phe Ile Ala Ser
          115           120          125

Val Ser Asp Gln His Gly Val Val Tyr Ile Thr Glu Asn Lys Asn Lys

```
              130                     135                        140


         Thr Val Val Ile Pro Cys Leu Gly Ser Ile Ser Asn Leu Asn Val Ser
         145                 150                 155                 160


         Leu Cys Ala Arg Tyr Pro Glu Lys Arg Phe Val Pro Asp Gly Asn Arg
                         165                 170                 175


         Ile Ser Trp Asp Ser Lys Lys Gly Phe Thr Ile Pro Ser Tyr Met Ile
                     180                 185                 190


         Ser Tyr Ala Gly Met Val Phe Cys Glu Ala Lys Ile Asn Asp Glu Ser
             195                 200                 205


         Tyr Gln Ser Ile Met Tyr Ile Val Val Val Gly Tyr Arg Ile Tyr
             210                 215                 220


         Asp Val Val Leu Ser Pro Ser His Gly Ile Glu Leu Ser Val Gly Glu
         225                 230                 235                 240


         Lys Leu Val Leu Asn Cys Thr Ala Arg Thr Glu Leu Asn Val Gly Ile
                         245                 250                 255


         Asp Phe Asn Trp Glu Tyr Pro Ser Ser Lys His Gln His Lys Lys Leu
                     260                 265                 270


         Val Asn Arg Asp Leu Lys Thr Gln Ser Gly Ser Glu Met Lys Lys Phe
                     275                 280                 285


         Leu Ser Thr Leu Thr Ile Asp Gly Val Thr Arg Ser Asp Gln Gly Leu
                     290                 295                 300


         Tyr Thr Cys Ala Ala Ser Ser Gly Leu Met Thr Lys Lys Asn Ser Thr
         305                 310                 315                 320


         Phe Val Arg Val His Glu Lys Pro Phe Val Ala Phe Gly Ser Gly Met
                         325                 330                 335


         Glu Ser Leu Val Glu Ala Thr Val Gly Glu Arg Val Arg Ile Pro Ala
                     340                 345                 350


         Lys Tyr Leu Gly Tyr Pro Pro Pro Glu Ile Lys Trp Tyr Lys Asn Gly
                     355                 360                 365


         Ile Pro Leu Glu Ser Asn His Thr Ile Lys Ala Gly His Val Leu Thr
             370                 375                 380
```

```
Ile Met Glu Val Ser Glu Arg Asp Thr Gly Asn Tyr Thr Val Ile Leu
385             390             395             400

Thr Asn Pro Ile Ser Lys Glu Lys Gln Ser His Val Val Ser Leu Val
                405             410             415

Val Tyr Val Pro Pro Gln Ile Gly Glu Lys Ser Leu Ile Ser Pro Val
            420             425             430

Asp Ser Tyr Gln Tyr Gly Thr Thr Gln Thr Leu Thr Cys Thr Val Tyr
            435             440             445

Ala Ile Pro Pro Pro His His Ile His Trp Tyr Trp Gln Leu Glu Glu
    450             455             460

Glu Cys Ala Asn Glu Pro Ser Gln Ala Val Ser Val Thr Asn Pro Tyr
465             470             475             480

Pro Cys Glu Glu Trp Arg Ser Val Glu Asp Phe Gln Gly Gly Asn Lys
            485             490             495

Ile Glu Val Asn Lys Asn Gln Phe Ala Leu Ile Glu Gly Lys Asn Lys
            500             505             510

Thr Val Ser Thr Leu Val Ile Gln Ala Ala Asn Val Ser Ala Leu Tyr
            515             520             525

Lys Cys Glu Ala Val Asn Lys Val Gly Arg Gly Glu Arg Val Ile Ser
    530             535             540

Phe His Val Thr Arg Gly Pro Glu Ile Thr Leu Gln Pro Asp Met Gln
545             550             555             560

Pro Thr Glu Gln Glu Ser Val Ser Leu Trp Cys Thr Ala Asp Arg Ser
            565             570             575

Thr Phe Glu Asn Leu Thr Trp Tyr Lys Leu Gly Pro Gln Pro Leu Pro
            580             585             590

Ile His Val Gly Glu Leu Pro Thr Pro Val Cys Lys Asn Leu Asp Thr
            595             600             605

Leu Trp Lys Leu Asn Ala Thr Met Phe Ser Asn Ser Thr Asn Asp Ile
    610             615             620

Leu Ile Met Glu Leu Lys Asn Ala Ser Leu Gln Asp Gln Gly Asp Tyr
625             630             635             640
```

```
Val Cys Leu Ala Gln Asp Arg Lys Thr Lys Lys Arg His Cys Val Val
                645                 650                     655

Arg Gln Leu Thr Val Leu Glu Arg Val Ala Pro Thr Ile Thr Gly Asn
                660                 665                 670

Leu Glu Asn Gln Thr Thr Ser Ile Gly Glu Ser Ile Glu Val Ser Cys
                675                 680                 685

Thr Ala Ser Gly Asn Pro Pro Pro Gln Ile Met Trp Phe Lys Asp Asn
                690             695                 700

Glu Thr Leu Val Glu Asp Ser Gly Ile Val Leu Lys Asp Gly Asn Arg
705                 710                 715                     720

Asn Leu Thr Ile Arg Arg Val Arg Lys Glu Asp Glu Gly Leu Tyr Thr
                725                 730                 735

Cys Gln Ala Cys Ser Val Leu Gly Cys Ala Lys Val Glu Ala Phe Phe
                740                 745                 750

Ile Ile Glu Gly Ala Gln Glu Lys Thr Asn Leu Glu Ile Ile Ile Leu
                755                 760                 765

Val Gly Thr Ala Val Ile Ala Met Phe Phe Trp Leu Leu Leu Val Ile
    770                 775                 780

Ile Leu Arg Thr Val Lys Arg Ala Asn Gly Gly Glu Leu Lys Thr Gly
785                 790                 795                     800

Tyr Leu Ser Ile Val Met Asp Pro Asp Glu Leu Pro Leu Asp Glu His
                805                 810                 815

Cys Glu Arg Leu Pro Tyr Asp Ala Ser Lys Trp Glu Phe Pro Arg Asp
                820                 825                 830

Arg Leu Lys Leu Gly Lys Pro Leu Gly Arg Gly Ala Phe Gly Gln Val
                835                 840                 845

Ile Glu Ala Asp Ala Phe Gly Ile Asp Lys Thr Ala Thr Cys Arg Thr
    850                 855                 860

Val Ala Val Lys Met Leu Lys Glu Gly Ala Thr His Ser Glu His Arg
865                 870                 875                     880

Ala Leu Met Ser Glu Leu Lys Ile Leu Ile His Ile Gly His His Leu
                885                 890                 895
```

40

```
Asn Val Val Asn Leu Leu Gly Ala Cys Thr Lys Pro Gly Gly Pro Leu
        900                 905                 910

Met Val Ile Val Glu Phe Cys Lys Phe Gly Asn Leu Ser Thr Tyr Leu
        915                 920                 925

Arg Ser Lys Arg Asn Glu Phe Val Pro Tyr Lys Thr Lys Gly Ala Arg
        930                 935                 940

Phe Arg Gln Gly Lys Asp Tyr Val Gly Ala Ile Pro Val Asp Leu Lys
945                 950                 955                 960

Arg Arg Leu Asp Ser Ile Thr Ser Ser Gln Ser Ser Ala Ser Ser Gly
                965                 970                 975

Phe Val Glu Glu Lys Ser Leu Ser Asp Val Glu Glu Glu Glu Ala Pro
        980                 985                 990

Glu Asp Leu Tyr Lys Asp Phe Leu Thr Leu Glu His Leu Ile Cys Tyr
        995                 1000                1005

Ser Phe Gln Val Ala Lys Gly Met Glu Phe Leu Ala Ser Arg Lys
        1010                1015                1020

Cys Ile His Arg Asp Leu Ala Ala Arg Asn Ile Leu Leu Ser Glu
        1025                1030                1035

Lys Asn Val Val Lys Ile Cys Asp Phe Gly Leu Ala Arg Asp Ile
        1040                1045                1050

Tyr Lys Asp Pro Asp Tyr Val Arg Lys Gly Asp Ala Arg Leu Pro
        1055                1060                1065

Leu Lys Trp Met Ala Pro Glu Thr Ile Phe Asp Arg Val Tyr Thr
        1070                1075                1080

Ile Gln Ser Asp Val Trp Ser Phe Gly Val Leu Leu Trp Glu Ile
        1085                1090                1095

Phe Ser Leu Gly Ala Ser Pro Tyr Pro Gly Val Lys Ile Asp Glu
        1100                1105                1110

Glu Phe Cys Arg Arg Leu Lys Glu Gly Thr Arg Met Arg Ala Pro
        1115                1120                1125

Asp Tyr Thr Thr Pro Glu Met Tyr Gln Thr Met Leu Asp Cys Trp
```

41

```
              1130                    1135                    1140


              His Gly Glu Pro Ser Gln Arg Pro Thr Phe Ser Glu Leu Val Glu
                  1145                    1150                    1155


              His Leu Gly Asn Leu Leu Gln Ala Asn Ala Gln Gln Asp Gly Lys
                  1160                    1165                    1170


              Asp Tyr Ile Val Leu Pro Ile Ser Glu Thr Leu Ser Met Glu Glu
                  1175                    1180                    1185


              Asp Ser Gly Leu Ser Leu Pro Thr Ser Pro Val Ser Cys Met Glu
                  1190                    1195                    1200


              Glu Glu Glu Val Cys Asp Pro Lys Phe His Tyr Asp Asn Thr Ala
                  1205                    1210                    1215


              Gly Ile Ser Gln Tyr Leu Gln Asn Ser Lys Arg Lys Ser Arg Pro
                  1220                    1225                    1230


              Val Ser Val Lys Thr Phe Glu Asp Ile Pro Leu Glu Glu Pro Glu
                  1235                    1240                    1245


              Val Lys Val Ile Pro Asp Asp Asn Gln Thr Asp Ser Gly Met Val
                  1250                    1255                    1260


              Leu Ala Ser Glu Glu Leu Lys Thr Leu Glu Asp Arg Thr Lys Leu
                  1265                    1270                    1275


              Ser Pro Ser Phe Gly Gly Met Val Pro Ser Lys Ser Arg Glu Ser
                  1280                    1285                    1290


              Val Ala Ser Glu Gly Ser Asn Gln Thr Ser Gly Tyr Gln Ser Gly
                  1295                    1300                    1305


              Tyr His Ser Asp Asp Thr Asp Thr Thr Val Tyr Ser Ser Glu Glu
                  1310                    1315                    1320


              Ala Glu Leu Leu Lys Leu Ile Glu Ile Gly Val Gln Thr Gly Ser
                  1325                    1330                    1335


              Thr Ala Gln Ile Leu Gln Pro Asp Ser Gly Thr Thr Leu Ser Ser
                  1340                    1345                    1350


              Pro Pro Val
                  1355
```

<210> 3
<211> 221
<212> PRT
<213> Homo sapiens

<400> 3

```
Met Pro Val Met Arg Leu Phe Pro Cys Phe Leu Gln Leu Leu Ala Gly
1               5               10              15

Leu Ala Leu Pro Ala Val Pro Pro Gln Gln Trp Ala Leu Ser Ala Gly
            20              25              30

Asn Gly Ser Ser Glu Val Glu Val Val Pro Phe Gln Glu Val Trp Gly
        35              40              45

Arg Ser Tyr Cys Arg Ala Leu Glu Arg Leu Val Asp Val Val Ser Glu
    50              55              60

Tyr Pro Ser Glu Val Glu His Met Phe Ser Pro Ser Cys Val Ser Leu
65              70              75              80

Leu Arg Cys Thr Gly Cys Cys Gly Asp Glu Asn Leu His Cys Val Pro
            85              90              95

Val Glu Thr Ala Asn Val Thr Met Gln Leu Leu Lys Ile Arg Ser Gly
        100             105             110

Asp Arg Pro Ser Tyr Val Glu Leu Thr Phe Ser Gln His Val Arg Cys
        115             120             125

Glu Cys Arg His Ser Pro Gly Arg Gln Ser Pro Asp Met Pro Gly Asp
    130             135             140

Phe Arg Ala Asp Ala Pro Ser Phe Leu Pro Pro Arg Arg Ser Leu Pro
145             150             155             160

Met Leu Phe Arg Met Glu Trp Gly Cys Ala Leu Thr Gly Ser Gln Ser
            165             170             175

Ala Val Trp Pro Ser Ser Pro Val Pro Glu Glu Ile Pro Arg Met His
            180             185             190

Pro Gly Arg Asn Gly Lys Lys Gln Gln Arg Lys Pro Leu Arg Glu Lys
        195             200             205

Met Lys Pro Glu Arg Cys Gly Asp Ala Val Pro Arg Arg
    210             215             220
```

43

**Description**

The present invention provides methods for improving the treatment effect of a chemotherapy regimen of a patient suffering from breast cancer, in particular locally advanced, recurrent or metastatic HER-2 negative breast cancer, by adding bevacizumab (Avastin®) to a chemotherapy regimen by determining the expression level, in particular the blood plasma expression level, of one or more of VEGFA, VEGFR2 and PLGF relative to control levels of patients diagnosed with breast cancer, in particular locally advanced, recurrent or metastatic HER-2 negative breast cancer. In particular, the present invention provides methods of improving the treatment effect, wherein the treatment effect is the progression-free survival of the patient. The present invention further provides for methods for assessing the sensitivity or responsiveness of a patient to bevacizumab (Avastin®) in combination with a chemotherapy regimen, by determining the expression level, in particular the blood plasma expression level, of one or more of VEGFA, VEGFR2 and PLGF relative to control levels in patients diagnosed with breast cancer, in particular locally advanced, recurrent or metastatic HER-2 negative breast cancer.

Accordingly, the present invention relates to the identification and selection of biomarkers of breast cancer, or locally advanced, recurrent or metastatic HER-2 negative breast cancer, that correlate with sensitivity or responsiveness to angiogenesis inhibitors, *e.g.,* bevacizumab (Avastin®), in combination with chemotherapeutic regimens, such as or docetaxel therapy. In this respect, the invention relates to the use of (a) blood plasma specific expression profile(s) of one or more of VEGFA, VEGFR2 and PLGF relative to controls established in patients diagnosed with breast cancer, in particular locally advanced, recurrent or metastatic HER-2 negative breast cancer, to identify patients sensitive or responsive to the addition of angiogenesis inhibitors, *e.g.,* bevacizumab (Avastin®), to standard chemotherapies. The invention further relates to methods for improving the treatment effect, in particular, the progression-free survival of a patient suffering from breast cancer, in particular locally advanced, recurrent or metastatic HER-2 negative breast cancer, by the addition of angiogenesis inhibitors, *e.g.,* bevacizumab (Avastin®), to standard chemotherapies, *e.g.,* docetaxel therapy, by determining (a) blood plasma specific expression level(s) of one or more of VEGFA, VEGFR2 and PLGF relative to control(s) in patients diagnosed with breast cancer, in particular locally advanced, recurrent or metastatic HER-2 negative breast cancer. The invention further provides for kits and compositions for identification of patients sensitive or responsive to angiogenesis inhibitors, in particular, bevacizumab (Avastin®), determined and defined in accordance with the methods of the present invention.

Angiogenesis is necessary for cancer development, regulating not only primary tumor size and growth but also impacting invasive and metastatic potential. Accordingly, the mechanisms mediating angiogenic processes have been investigated as potential targets for directed anti-cancer therapies. Early in the study of angiogenic modulators, the vascular endothelial growth factor (VEGF) signalling pathway was discovered to preferentially regulate angiogenic activity in multiple cancer types. This factor signals through VEGF Receptor 2 (VEGFR-2), the major VEGF signalling receptor that mediates sprouting angiogenesis. Multiple therapeutics have been developed to modulate this pathway at various points. These therapies include, among others, bevacizumab, sunitinib, sorafenib and vatalanib. Although the use of angiogenic inhibitors in the clinic has shown success, not all patients respond or fail to fully respond to angiogenesis inhibitor therapy. The mechanism(s) underlying such incomplete response is unknown. Therefore, there is an increasing need for the identification of patient subgroups sensitive or responsive to anti-angiogenic cancer therapy.

While a number of angiogenesis inhibitors are known, the most prominent angiogenesis inhibitor is bevacizumab (Avastin®). Bevacizumab is a recombinant humanized monoclonal IgG1 antibody that specifically binds and blocks the biological effects of VEGF (vascular endothelial growth factor). VEGF is a key driver of tumor angiogenesis - an essential process required for tumor growth and metastasis, *i.e.,* the dissemination of the tumor to other parts of the body. Avastin® is approved in Europe for the treatment of the advanced stages of four common types of cancer: colorectal cancer, breast cancer, non-small cell lung cancer (NSCLC) and kidney cancer, which collectively cause over 2.5 million deaths each year. In the United States, Avastin® was the first anti-angiogenesis therapy approved by the FDA, and it is now approved for the treatment of five tumor types: colorectal cancer, non-small cell lung cancer, breast cancer, brain (glioblastoma) and kidney (renal cell carcinoma). Over half a million patients have been treated with Avastin so far, and a comprehensive clinical program with over 450 clinical trials is investigating the further use of Avastin in the treatment of multiple cancer types (including colorectal, breast, non-small cell lung, brain, gastric, ovarian and prostate) in different settings (*e.g.*, advanced or early stage disease). Importantly, Avastin® has shown promise as a co-therapeutic, demonstrating efficacy when combined with a broad range of chemotherapies and other anti-cancer treatments. Phase-III studies have been published demonstrating the beneficial effects of combining bevacizumab with standard chemotherapeutic regimens (see, *e.g.,* Saltz et al., 2008, J. Clin. Oncol., 26:2013-2019; Yang et al., 2008, Clin. Cancer Res., 14:5893-5899; Hurwitz et al., 2004, N. Engl. J. Med., 350:2335-2342). However, as in previous studies of angiogenic inhibitors, some of these phase-III studies have shown that a portion of patients experience incomplete response to the addition of bevacizumab (Avastin®) to their chemotherapeutic regimens.

Accordingly, there is a need for methods of determining those patients that respond to or are likely to respond to combination therapies comprising angiogenesis inhibitors, in particular, bevacizumab (Avastin®). Thus, the technical problem underlying the present invention is the provision of methods and means for the identification of (a) patient(s) suffering from or prone to suffer from breast cancer, in particular locally advanced, recurrent or metastatic HER-2 negative breast cancer, who may benefit from the addition of angiogenesis inhibitors, in particular, bevacizumab (Avastin®), to chemotherapeutic therapies, *e.g.,* docetaxel therapy.

The technical problem is solved by provision of the embodiments characterized in the claims.

The present invention, therefore, provides a method for improving the treatment effect of a chemotherapy regimen of a patient suffering from breast cancer by adding bevacizumab to said chemotherapy regimen, said method comprising:

(a) determining the protein expression level of one or more of VEGFA, VEGFR2 and PLGF in a patient sample; and
(b) administering bevacizumab in combination with a chemotherapy regimen to the patient having an increased expression level of one or more of VEGFA, VEGFR2 and PLGF relative to control expression levels determined in patients diagnosed with breast cancer.

The present invention relates to a method for improving the treatment effect of a chemotherapy regimen of a patient suffering from breast cancer by adding bevacizumab to the chemotherapy regimen, said method comprising:

(a) obtaining a sample from said patient;
(b) determining the protein expression level of one or more of VEGFA, VEGFR2 and PLGF; and
(c) administering bevacizumab in combination with a chemotherapy regimen to the patient having an increased expression level of one or more of VEGFA, VEGFR2 and PLGF relative to control expression levels determined in patients diagnosed with breast cancer.

The present invention relates to a method for improving the progression free survival of a patient suffering from breast cancer by adding bevacizumab to a chemotherapy regimen, said method comprising:

(a) determining the protein expression level of one or more of VEGFA, VEGFR2 and PLGF in a patient sample; and
(b) administering bevacizumab in combination with the chemotherapy regimen to the patient having an increased expression level of one or more of VEGFA, VEGFR2 and PLGF relative to control expression levels determined in patients diagnosed with breast cancer.

The present invention relates to a method for improving the progression free survival of a patient suffering from breast cancer by adding bevacizumab to a chemotherapy regimen, said method comprising:

(a) obtaining a sample from said patient;
(b) determining the protein expression level of one or more of VEGFA, VEGFR2 and PLGF; and
(c) administering bevacizumab in combination with the chemotherapy regimen to the patient having an increased expression level of one or more of VEGFA, VEGFR2 and PLGF relative to control expression levels determined in patients diagnosed with breast cancer.

The present invention relates to a method for improving the progression free survival of a patient suffering from locally advanced, recurrent or metastatic HER-2 negative breast cancer by adding bevacizumab to a chemotherapy regimen, said method comprising:

(a) determining the protein expression level of one or more of VEGFA, VEGFR2 and PLGF in a patient sample; and
(b) administering bevacizumab in combination with the chemotherapy regimen to the patient having an increased expression level of one or more of VEGFA, VEGFR2 and PLGF relative to control expression levels determined in patients diagnosed with said breast cancer,

wherein the chemotherapy regimen comprises docetaxel therapy.

The present invention relates to a method for improving the progression free survival of a patient suffering from locally advanced, recurrent or metastatic HER-2 negative breast cancer by adding bevacizumab to a chemotherapy regimen, said method comprising:

(a) obtaining a sample from said patient;
(b) determining the protein expression level of one or more of VEGFA, VEGFR2 and PLGF; and
(c) administering bevacizumab in combination with the chemotherapy regimen to the patient having an increased expression level of one or more of VEGFA, VEGFR2 and PLGF relative to control expression levels determined in patients diagnosed with said breast cancer,

wherein the chemotherapy regimen comprises docetaxel therapy.

The present invention relates to a method for improving the progression free survival of a patient suffering from breast cancer by adding bevacizumab to a chemotherapy regimen, said method comprising:

(a) determining the protein expression level of VEGFA or VEGFR2 in a patient sample; and
(b) administering bevacizumab in combination with the chemotherapy regimen to the patient having an increased expression level of VEGFA or VEGFR2 relative to control expression levels determined in patients diagnosed with breast cancer.

The present invention relates to a method for improving the progression free survival of a patient suffering from breast cancer by adding bevacizumab to a chemotherapy regimen, said method comprising:

(a) obtaining a sample from said patient;
(b) determining the protein expression level of VEGFA or VEGFR2; and
(c) administering bevacizumab in combination with the chemotherapy regimen to the patient having an increased expression level of VEGFA or VEGFR2 relative to control expression levels determined in patients diagnosed with breast cancer.

The present invention relates to a method for improving the progression free survival of a patient suffering from locally advanced, recurrent or metastatic HER-2 negative breast cancer by adding bevacizumab to a chemotherapy regimen, said method comprising:

(a) determining the protein expression level of VEGFA or VEGFR2 in a patient sample; and
(b) administering bevacizumab in combination with the chemotherapy regimen to the patient having an increased expression level of VEGFA or VEGFR2 relative to control expression levels determined in patients diagnosed with said breast cancer,

wherein the chemotherapy regimen comprises docetaxel therapy.

The present invention relates to a method for improving the progression free survival of a patient suffering from locally advanced, recurrent or metastatic HER-2 negative breast cancer by adding bevacizumab to a chemotherapy regimen, said method comprising:

(a) obtaining a sample from said patient;
(b) determining the protein expression level of VEGFA or VEGFR2; and
(c) administering bevacizumab in combination with the chemotherapy regimen to the patient having an increased expression level of one or more of VEGFA or VEGFR2 relative to control expression levels determined in patients diagnosed with said breast cancer,

wherein the chemotherapy regimen comprises docetaxel therapy.

The invention provides a method for improving the treatment effect of a chemotherapy regimen of a patient suffering from breast cancer by adding bevacizumab to a chemotherapy regimen, said method comprising:

(a) determining the protein expression level of VEGFA, VEGFR2 and PLGF in a patient sample; and
(b) administering bevacizumab in combination with a chemotherapy regimen to the patient having an increased combined expression level of VEGFA, VEGFR2 and PLGF relative to a control combined expression level determined in patients diagnosed with breast cancer.

The breast cancer may be locally advanced, recurrent or metastatic HER-2 negative breast cancer. The chemotherapy regimen may comprise taxane therapy, such as docetaxel or paclitaxel therapy.

Accordingly, the invention relates to a method for improving the treatment effect of a chemotherapy regimen of a patient suffering from breast cancer by adding bevacizumab to a chemotherapy regimen, said method comprising:

(a) obtaining a sample from said patient;
(b) determining the protein expression level of VEGFA, VEGFR2 and PLGF; and
(c) administering bevacizumab in combination with a chemotherapy regimen to the patient having an increased combined expression level of VEGFA, VEGFR2 and PLGF relative to a control combined expression level determined in patients diagnosed with breast cancer.

The breast cancer may be locally advanced, recurrent or metastatic HER-2 negative breast cancer. The chemotherapy regimen may comprise taxane therapy, such as docetaxel or paclitaxel therapy.

The invention provides a method for improving the progression free survival of a patient suffering from breast cancer by adding bevacizumab to a chemotherapy regimen, said method comprising:

(a) determining the protein expression level of VEGFA, VEGFR2 and PLGF in a patient sample; and
(b) administering bevacizumab in combination with a chemotherapy regimen to the patient having an increased combined expression level of VEGFA, VEGFR2 and PLGF relative to a control combined expression level determined in patients diagnosed with breast cancer.

The breast cancer may be locally advanced, recurrent or metastatic HER-2 negative breast cancer. The chemotherapy regimen may comprise taxane therapy, such as docetaxel or paclitaxel therapy.

Accordingly, the invention relates to a method for improving the progression free survival of a patient suffering from breast cancer by adding bevacizumab to a chemotherapy regimen, said method comprising:

(a) obtaining a sample from said patient;
(b) determining the protein expression level of VEGFA, VEGFR2 and PLGF; and
(c) administering bevacizumab in combination with a chemotherapy regimen to the patient having an increased combined expression level of VEGFA, VEGFR2 and PLGF relative to a control combined expression level determined in patients diagnosed with breast cancer.

The breast cancer may be locally advanced, recurrent or metastatic HER-2 negative breast cancer. The chemotherapy regimen may comprise taxane therapy, such as docetaxel or paclitaxel therapy.

The invention, therefore, relates to a method for improving the progression free survival of a patient suffering from locally advanced, recurrent or metastatic HER-2 negative breast cancer by adding bevacizumab to a chemotherapy regimen, said method comprising:

(a) determining the protein expression level of VEGFA, VEGFR2 and PLGF in a patient sample; and
(b) administering bevacizumab in combination the chemotherapy regimen to the patient having an increased combined expression level of VEGFA, VEGFR2 and PLGF relative to a control combined expression level determined in patients diagnosed with said breast cancer,

wherein the chemotherapy regimen comprises docetaxel therapy.

The invention relates to a method for improving the progression free survival of a patient suffering from locally advanced, recurrent or metastatic HER-2 negative breast cancer by adding bevacizumab to a chemotherapy regimen, said method comprising:

(a) obtaining a sample from said patient;
(b) determining the protein expression level of VEGFA, VEGFR2 and PLGF; and
(c) administering bevacizumab in combination with the chemotherapy regimen to the patient having an increased combined expression level of VEGFA, VEGFR2 and PLGF relative to a control combined expression level determined in patients diagnosed with said cancer,

wherein the chemotherapy regimen comprises docetaxel therapy.

The invention provides a method for improving the progression free survival of a patient suffering from breast cancer by adding bevacizumab to a chemotherapy regimen, said method comprising:

(a) determining the protein expression level of VEGFA and VEGFR2 in a patient sample; and
(b) administering bevacizumab in combination with the chemotherapy regimen to the patient having an increased combined expression level of VEGFA and VEGFR2 relative to a control combined expression level determined in patients diagnosed with breast cancer.

The breast cancer may be locally advanced, recurrent or metastatic HER-2 negative breast cancer. The chemotherapy regimen may comprise taxane therapy, such as docetaxel or paclitaxel therapy. The dose of the bevacizumab administered may be low or high dose bevacizumab. As shown in the appended illustrative example, the combined expression level of VEGFA and VEGFR2 was particularly predictive in patients administered low dose bevacizumab.

Accordingly, the invention relates to a method for improving the progression free survival of a patient suffering from locally advanced, recurrent or metastatic HER-2 negative breast cancer by adding bevacizumab to a chemotherapy regimen, said method comprising:

(a) obtaining a sample from said patient;
(b) determining the protein expression level of VEGFA and VEGFR2; and
(c) administering bevacizumab in combination with the chemotherapy regimen to the patient having an increased combined expression level of VEGFA and VEGFR2 relative to a control combined expression level determined in patients diagnosed with said breast cancer.

The chemotherapy regimen may comprise taxane therapy, such as docetaxel or paclitaxel therapy. The dose of the bevacizumab administered may be low or high dose bevacizumab. As shown in the appended illustrative example, the combined expression level of VEGFA and VEGFR2 was particularly predictive in patients administered low dose bevacizumab.

The invention, therefore relates to a method for improving the progression free survival of a patient suffering from locally advanced, recurrent or metastatic HER-2 negative breast cancer by adding bevacizumab to a chemotherapy regimen, said method comprising:

(a) determining the protein expression level of VEGFA and VEGFR2 in a patient sample; and
(b) administering bevacizumab in combination with the chemotherapy regimen to the patient having an increased combined expression level of VEGFA and VEGFR2 relative to a control combined expression level determined in patients diagnosed with said breast cancer,

wherein the chemotherapy regimen comprises docetaxel therapy. The dose of bevacizumab administered may be low or high dose bevacizumab. As shown in the appended illustrative example, the combined expression level of VEGFA and VEGFR2 was particularly predictive in patients administered low dose bevacizumab.

The invention relates to a method for improving the progression free survival of a patient suffering from locally advanced, recurrent or metastatic HER-2 negative breast cancer by adding bevacizumab to a chemotherapy regimen, said method comprising:

(a) obtaining a sample from said patient;
(b) determining the protein expression level of VEGFA and VEGFR2; and
(c) administering bevacizumab in combination with the chemotherapy regimen to the patient having an increased combined expression level of VEGFA and VEGFR2 relative to a control combined expression level determined in patients diagnosed with said breast cancer,

wherein the chemotherapy regimen comprises docetaxel therapy. The dose of bevacizumab administered may be low or high dose bevacizumab. As shown in the appended illustrative example, the combined expression level of VEGFA and VEGFR2 was particularly predictive in patients administered low dose bevacizumab.

The present invention provides a method for improving the progression-free survival of a patient suffering from breast cancer by adding bevacizumab to a chemotherapy regimen, said method comprising:

(a) determining the protein expression level of VEGFA and PLGF in a patient sample; and
(b) administering bevacizumab in combination with the chemotherapy regimen to the patient having an increased combined expression level of VEGFA and PLGF relative to a combined control expression level determined in patients diagnosed with breast cancer.

The breast cancer may be locally advanced, recurrent or metastatic HER-2 negative breast cancer. The chemotherapy regimen may comprise taxane therapy, such as docetaxel or paclitaxel therapy. The dose of the bevacizumab administered may be low dose or high dose bevacizumab.

Accordingly, the present invention relates to a method for improving the progression free survival of a patient suffering from breast cancer by adding bevacizumab to a chemotherapy regimen, said method comprising:

(a) obtaining a sample from said patient;
(b) determining the protein expression level of VEGFA and PLGF; and
(c) administering bevacizumab in combination with the chemotherapy regimen to the patient having an increased combined expression level of VEGFA and PLGF relative to a control combined expression level determined in patients diagnosed with breast cancer.

The breast cancer may be locally advanced, recurrent or metastatic HER-2 negative breast cancer. The chemotherapy regimen may comprise taxane therapy, such as docetaxel or paclitaxel therapy. The dose of the bevacizumab administered may be low dose or high dose bevacizumab.

The invention, therefore, relates to a method for improving the progression free survival of a patient suffering from locally advanced, recurrent or metastatic HER-2 negative breast cancer by adding bevacizumab to a chemotherapy regimen, said method comprising:

(a) determining the protein expression level of VEGFA and PLGF in a patient sample; and
(b) administering bevacizumab in combination with the chemotherapy regimen to the patient having an increased combined expression level of VEGFA and PLGF relative to a combined control expression level determined in patients diagnosed with said breast cancer,

wherein the chemotherapy regimen comprises docetaxel therapy. The dose of the bevacizumab administered may be low dose or high dose bevacizumab.

Accordingly, the present invention relates to a method for improving the progression free survival of a patient suffering from locally advanced, recurrent or metastatic HER-2 negative breast cancer by adding bevacizumab to a chemotherapy regimen, said method comprising:

(a) obtaining a sample from said patient;
(b) determining the protein expression level of VEGFA and PLGF; and
(c) administering bevacizumab in combination with the chemotherapy regimen to the patient having an increased combined expression level of VEGFA and PLGF relative to a control combined expression level determined in patients diagnosed with said breast cancer,

wherein the chemotherapy regimen comprises docetaxel therapy. The dose of the bevacizumab administered may be low dose or high dose bevacizumab.

The present invention provides an in vitro method for the identification of a patient responsive to or sensitive to the addition of bevacizumab treatment to a chemotherapy regimen, said method comprising determining the protein expression level of one or more of VEGFA, VEGFR2 and PLGF in a sample from a patient suffering from, suspected to suffer from or being prone to suffer from breast cancer, in particular locally advanced, recurrent or metastatic HER-2 negative breast cancer, whereby an increased expression level of one or more of VEGFA, VEGFR2 and PLGF relative to control expression levels determined in patients suffering from breast cancer, in particular locally advanced, recurrent or metastatic HER-2 negative breast cancer, is indicative of a sensitivity of the patient to the addition of bevacizumab to said chemotherapy regimen. The chemotherapy regimen may comprise taxane therapy, such as docetaxel or paclitaxel therapy.

Accordingly, the present invention relates to an in vitro method for the identification of a patient responsive to or sensitive to the addition of bevacizumab treatment to a chemotherapy regimen, said method comprising:

(a) obtaining a sample from a patient suffering from, suspected to suffer from or being prone to suffer from breast cancer, locally advanced, recurrent or metastatic HER-2 negative breast cancer; and
(b) determining the protein expression level of one or more of VEGFA, VEGFR2 and PLGF;

whereby an increased expression level of one or more of VEGFA, VEGFR2 and PLGF relative to control expression levels determined in patients suffering from breast cancer, in particular locally advanced, recurrent or metastatic HER-2 negative breast cancer, is indicative of a sensitivity of the patient to the addition of bevacizumab to said chemotherapy regimen. The chemotherapy regimen may comprise taxane therapy, such as docetaxel or paclitaxel therapy.

The present invention provides an in vitro method for the identification of a patient that is responsive to or sensitive to the addition of bevacizumab treatment to a chemotherapy regimen, said method comprising determining the protein expression level of VEGFA and VEGFR2 in a sample from a patient suffering from, suspected to suffer from or being prone to suffer from locally advanced, recurrent or metastatic HER-2 negative breast cancer, whereby a combined increased expression level of VEGFA and VEGFR2 relative to control combined expression levels determined in patients suffering from locally advanced, recurrent or metastatic HER-2 negative breast cancer is indicative of a sensitivity of the patient to the addition of bevacizumab to said chemotherapy regimen. The chemotherapy regimen may comprise taxane therapy, such as docetaxel or paclitaxel therapy.

The present invention provides an in vitro method for the identification of a patient that is responsive to or sensitive to the addition of bevacizumab treatment to a chemotherapy regimen, said method comprising determining the protein expression level of VEGFA and PLGF in a sample from a patient suffering from, suspected to suffer from or being prone to suffer from locally advanced, recurrent or metastatic HER-2 negative breast cancer, whereby a combined increased expression level of VEGFA and PLGF relative to control combined levels determined in patients suffering from locally advanced, recurrent or metastatic HER-2 negative breast cancer is indicative of a sensitivity of the patient to the addition of bevacizumab to said chemotherapy regimen. The chemotherapy regimen may comprise taxane therapy, such as docetaxel or paclitaxel therapy.

Accordingly, the present invention solves the identified technical problem in that it was surprisingly shown that the blood plasma specific expression levels of one or more of VEGFA, VEGFR2 and PLGF in a given patient, relative to control levels determined in patients diagnosed with breast cancer, in particular locally advanced, recurrent or metastatic HER-2 negative breast cancer, correlate with treatment effect in those patients administered an angiogenesis inhibitor in combination with a chemotherapy regimen. Specifically, variations in the protein expression levels of VEGFA, VEGFR2 and/or PLGF were surprisingly identified as markers/predictors for the improved progression-free survival of locally advanced, recurrent or metastatic HER-2 negative breast cancer patients in response to the addition of bevacizumab (Avastin®) to the chemotherapy regimen of docetaxel therapy. Patients exhibiting a response or sensitivity to the addition of bevacizumab (Avastin®) to chemotherapy regimens were identified to have an increased protein expression level of one or more VEGFA, VEGFR2 and PLGF relative to control expression levels established in samples obtained from patients diagnosed with breast cancer, in particular locally advanced, recurrent or metastatic HER-2 negative breast cancer. The terms "marker" and "predictor" can be used interchangeably and refer to the expression levels of one or more of VEGFA, VEGFR2 and PLGF as described herein. The invention also encompasses the use of the terms "marker" and "predictor" to refer to a combination of any two or more of the blood plasma expression levels of VEGFA, VEGFR2 and PLGF.

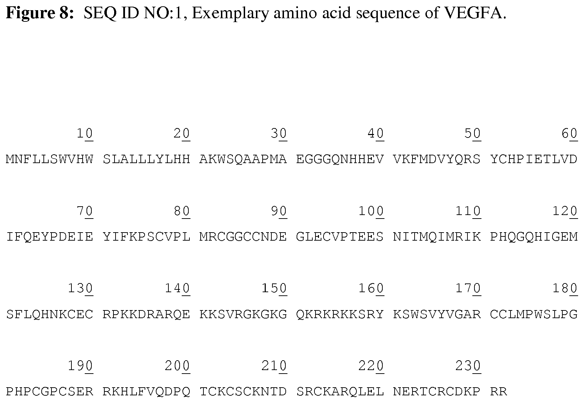

In the context of the present invention, "VEGFA" refers to vascular endothelial growth factor protein A, exemplified by SEQ ID NO:1, shown in FIGURE 8 (Swiss Prot Accession Number P15692, Gene ID (NCBI): 7422). The term "VEGFA" encompasses the protein having the amino acid sequence of SEQ ID NO:1 as well as homologues and isoforms thereof. The term "VEGFA" also encompasses the known isoforms, *e.g.,* splice isoforms, of VEGFA, *e.g.,* $VEGF_{111}$, $VEGF_{121}$, $VEGF_{145}$, $VEGF_{165}$, $VEGF_{189}$ and $VEGF_{206}$, as well as variants, homologues and isoforms thereof, including the 110- amino acid human vascular endothelial cell growth factor generated by plasmin cleavage of $VEGF_{165}$ as described in Ferrara Mol. Biol. Cell 21:687 (2010) and Leung et al. Science 246:1306 (1989), and Houck et al. Mol. Endocrin. 5:1806 (1991). In a particular embodiment of the present invention, "VEGFA" refers to $VEGF_{121}$ and/or $VEGF_{110}$. In a particular embodiment of the present invention, "VEGFA" refers to $VEGF_{111}$. In the context of the invention, the term "VEGFA" also encompasses proteins having at least 85%, at least 90% or at least 95% homology to the amino acid sequence of SEQ ID NO:1, or to the amino acid sequences of the variants and/or homologues thereof, as well as fragments of the sequences, provided that the variant proteins (including isoforms), homologous proteins and/or fragments are recognized by one or more VEGFA specific antibodies, such as antibody clone 3C5 and 26503, which are available from Bender RELIATech and R&D Systems, respectively and A4.6.1 as described in Kim et al., Growth Factors 7(1): 53-64 (1992). In the context of the invention, the term "isoform" of VEGF or VEGF-A refers to both splice isoforms and forms generated by enzymatic cleavage (e.g., plasmin).

In one embodiment, "VEGFA" refers to unmodified VEGF. In the context of the present invention "unmodified" VEGF relates to the unmodified amino acid sequence of VEGF, its isoforms and its cleavage products. Unmodified VEGF can e.g. be produced synthetically or preferably recombinantly in prokaryotic expression systems, e.g. in E. coli. Unmodified VEGF does e.g. not carry a posttranslational modification, like a glycosylation. In the context of the invention, the term "unmodified VEGF-A" also encompasses variants and/or homologues thereof, as well as fragments of VEGF-A, provided that the variant proteins (including isoforms), homologous proteins and/or fragments are recognized by an unmodified VEGF-A specific antibodies, such as antibody clone 3C5, which is available from RELIATech GmbH, Wolfenbüttel, Germany.

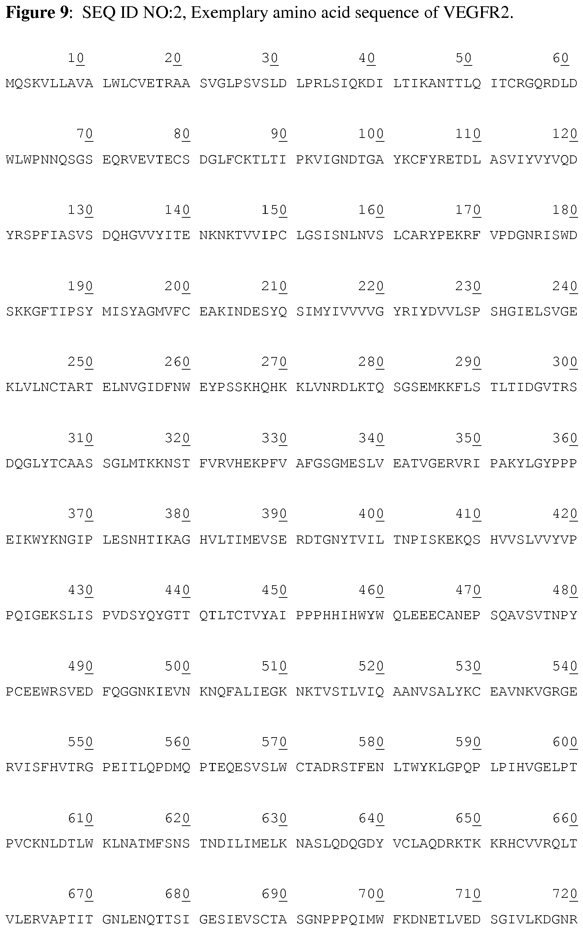

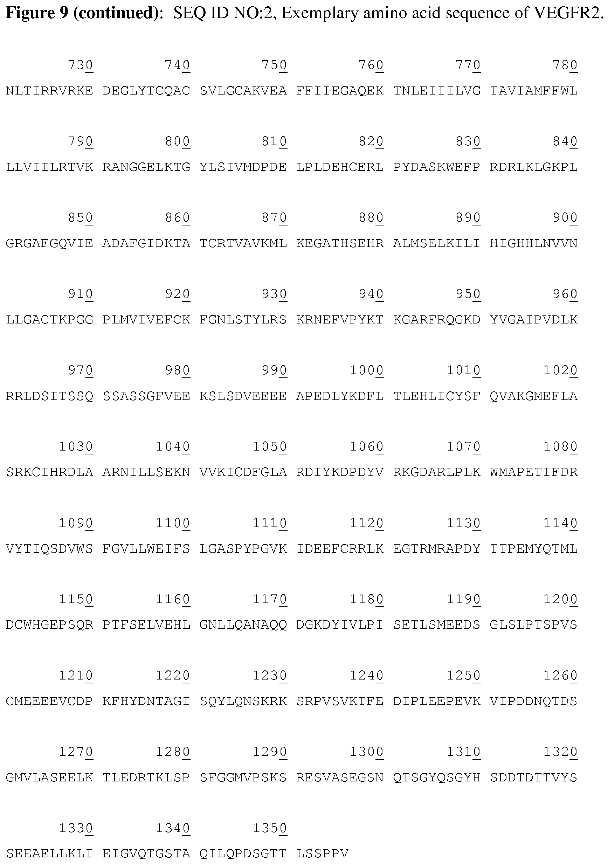

In the context of the present invention, "VEGFR2" refers to vascular endothelial growth factor receptor 2, exemplified by SEQ ID NO:2, shown in FIGURE 9 (Swiss Prot Accession Number P35968, Gene ID (NCBI): 3791). The term "VEGFR2" encompasses the protein having the amino acid sequence of SEQ ID NO:2 as well as homologues and isoforms thereof. In the context of the invention, the term "VEGFR2" also encompasses proteins having at least 85%, at least 90% or at least 95% homology to the amino acid sequence of SEQ ID NO:2, or to the amino acid sequences of the variants and/or homologues thereof, as well as fragments of the sequences, provided that the variant proteins (including isoforms), homologous proteins and/or fragments are recognized by one or more VEGFR2 specific antibodies, such as antibody clone 89115 and 89109, which are available from R&D Systems.

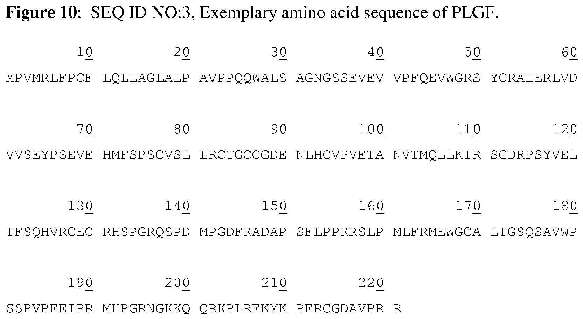

In the context of the present invention, "PLGF" refers to placental growth factor exemplified by SEQ ID NO:3, shown in FIGURE 10 (Swiss Prot Accession Number P49763, Gene ID (NCBI): 5228). The term "PLGF" encompasses the protein having the amino acid sequence of SEQ ID NO:3 as well as homologues and isoforms thereof. In the context of the invention, the term "PLGF" also encompasses proteins having at least 85%, at least 90% or at least 95% homology to the amino acid sequence of SEQ ID NO:3, or to the amino acid sequences of the variants and/or homologues thereof, as well as fragments of the sequences, provided that the variant proteins (including isoforms), homologous proteins and/or fragments are recognized by one or more PLGF specific antibodies, such as antibody clone 2D6D5 and 6A11D2, which are available from Roche Diagnostics GmbH.

Accordingly, the present invention encompasses the determination of expression levels of proteins including, but not limited to, the amino acid sequences as described herein. In this context the invention encompasses the detection of homologues, variants and isoforms of one or more of VEGFA, VEGFR2 and PLGF; said isoforms or variants may, inter alia, comprise allelic variants or splice variants. Also envisaged is the detection of proteins that are homologous to one or more of VEGFA, VEGFR2 and PLGF as herein described, or a fragment thereof, e.g., having at least 60%, 70%, 80%, 90%, 95%, 96%, 97%, 98% or 99% sequence identity to the amino acid sequence of SEQ ID NO:1, SEQ ID NO:2 or SEQ ID NO:3 or a fragment thereof. Alternatively or additionally, the present invention encompasses detection of the expression levels of proteins encoded by nucleic acid sequences, or fragments thereof, that are at least at least 60%, 70%, 80%, 90%, 95%, 96%, 97%, 98% or 99% identical to a nucleic acid sequence encoding SEQ ID NO:1, SEQ ID NO:2 or SEQ ID NO:3 or a fragment, variant or isoform thereof. In this context, the term "variant" means that the VEGFA, VEGFR2 and/or PLGF amino acid sequence, or the nucleic acid sequence encoding said amino acid sequence, differs from the distinct sequences identified by SEQ ID NOs:1, SEQ ID NO:2 or SEQ ID NO:3 and/or available under the above-identified Swiss Prot Accession numbers, by mutations, e.g., deletion, additions, substitutions, inversions etc. In addition, the term "homologue" references molecules having at least 60%, more preferably at least 80% and most preferably at least 90% sequence identity to one or more of the polypeptides as shown in SEQ ID NOs:1, SEQ ID NO:2 or SEQ ID NO:3, or (a) fragment(s) thereof.

In order to determine whether an amino acid or nucleic acid sequence has a certain degree of identity to an amino acid or nucleic acid sequence as herein described, the skilled person can use means and methods well known in the art, e.g. alignments, either manually or by using computer programs known in the art or described herein.

In accordance with the present invention, the term "identical" or "percent identity" in the context of two or more or amino acid or nucleic acid sequences, refers to two or more sequences or subsequences that are the same, or that have a specified percentage of amino acid residues or nucleotides that are the same (e.g., 60% or 65% identity, preferably, 70-95% identity, more preferably at least 95% identity with the amino acid sequences of, *e.g.,* SEQ ID NO:1, SEQ ID NO:2 or SEQ ID NO:3), when compared and aligned for maximum correspondence over a window of comparison, or over a designated region as measured using a sequence comparison algorithm as known in the art, or by manual alignment and visual inspection. Sequences having, for example, 60% to 95% or greater sequence identity are considered to be substantially identical. Such a definition also applies to the complement of a test sequence. Preferably the described identity exists over a region that is at least about 15 to 25 amino acids or nucleotides in length, more preferably, over a region that is about 50 to 100 amino acids or nucleotides in length. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

Although the FASTDB algorithm typically does not consider internal non-matching deletions or additions in sequences, *i.e.,* gaps, in its calculation, this can be corrected manually to avoid an overestimation of the % identity. CLUSTALW, however, does take sequence gaps into account in its identity calculations. Also available to those having skill in this art are the BLAST (Basic Local Alignment Search Tool) and BLAST 2.0 algorithms (Altschul, 1997, Nucl. Acids Res. 25:3389-3402; Altschul, 1993 J. Mol. Evol. 36:290-300; Altschul, 1990, J. Mol. Biol. 215:403-410). The BLASTN program for nucleic acid sequences uses as defaults a word length (W) of 11, an expectation (E) of 10, M=5, N=4, and a comparison of both strands. For amino acid sequences, the BLASTP program uses as defaults a word length (W) of 3, and an expectation (E) of 10. The BLOSUM62 scoring matrix (Henikoff (1989) PNAS 89:10915) uses alignments (B) of 50, expectation (E) of 10, M=5, N=4, and a comparison of both strands.

BLAST algorithms, as discussed above, produce alignments of both amino and nucleotide sequences to determine sequence similarity. Because of the local nature of the alignments, BLAST is especially useful in determining exact matches or in identifying similar sequences. The fundamental unit of BLAST algorithm output is the High-scoring Segment Pair (HSP). An HSP consists of two sequence fragments of arbitrary but equal lengths whose alignment is locally maximal and for which the alignment score meets or exceeds a threshold or cut-off score set by the user. The BLAST approach is to look for HSPs between a query sequence and a database sequence, to evaluate the statistical significance of any matches found, and to report only those matches which satisfy the user-selected threshold of significance. The parameter E establishes the statistically significant threshold for reporting database sequence matches. E is interpreted as the upper bound of the expected frequency of chance occurrence of an HSP (or set of HSPs) within the context of the entire database search. Any database sequence whose match satisfies E is reported in the program output.

Analogous computer techniques using BLAST may be used to search for identical or related molecules in protein or nucleotide databases such as GenBank or EMBL. This analysis is much faster than multiple membrane-based hybridizations. In addition, the sensitivity of the computer search can be modified to determine whether any particular match is categorized as exact or similar. The basis of the search is the product score which is defined as:

$$\frac{\%\ \text{sequence identity}\ \times\ \%\ \text{maximum BLAST score}}{100}$$

and takes into account both the degree of similarity between two sequences and the length of the sequence match. For example, with a product score of 40, the match will be exact within a 1-2% error; and at 70, the match will be exact. Similar molecules are usually identified by selecting those which show product scores between 15 and 40, although lower scores may identify related molecules. Another example for a program capable of generating sequence alignments is the CLUSTALW computer program (Thompson, 1994, Nucl. Acids Res. 2:4673-4680) or FASTDB (Brutlag, 1990, Comp. App. Biosci. 6:237-245), as is known in the art.

In the context of the herein described invention, the expression levels, in particular protein expression levels, of VEGFA, VEGFR2 and/or PLGF, may be considered separately, as individual markers, or in groups of two or more, as an expression profile or marker panel. In the context of the herein described invention an expression profile or marker panel wherein the expression profiles of two or more markers may be considered together may also be referred to as a combined expression level. For example, the expression levels of two or more markers may be added together and compared to a similarly determined control combined expression level. Therefore, the methods of the invention encompass determination of an expression profile, including a combined expression level, based on the expression level of one or more of the markers.

In the context of the herein described invention, and in accordance with the appended illustrative example, for consideration of VEGFA or VEGFR2 separately, the following values were used as the corresponding high or low expression value of the marker: High VEGFA ($\geq$125 pg/ml), Low VEGFA (<125 pg/ml), High VEGFR2 ($\geq$11 mg/ml) and Low VEGFR2 (<11 ng/ml). These levels were determined as the sample median, as per a prospective analysis plan. Additionally, optimized levels constituting the cut-off value between high and low expression of a particular marker may be determined by varying the cut-off until the subset of patients above and below the cut-off satisfy a relevant statistical optimality criterion. For example, an optimal cut-point may be chosen to maximize the differences in treatment Hazard Ratio between the subset above and below, or to maximize treatment effect in one sub-group, or any other relevant statistical criterion. The skilled person will, however, understand that the expression level of the particular marker and, therefore, what constitutes a high or low expression level may vary by patient and by patient population. Accordingly, the skilled person will understand that when using detection methods other than those described in the appended illustrative example and studying patients and patient populations other than those described in the appended illustrative example, what the skilled person considers a high and/or low expression level for a particular biomarker may vary from the values herein described. Given the methods herein described, the skilled person can determine what constitutes a high and/or low level of expression of a particular biomarker.

As the skilled artisan will appreciate there are many ways to use the measurements of two or more markers in order to improve the diagnostic question under investigation. In a quite simple, but nonetheless often effective approach, a positive result is assumed if a sample is positive for at least one of the markers investigated.

However, a combination of markers may also be evaluated. The values measured for markers of a marker panel (or a combined expression level), e.g. for VEGFA and VEGFR2 or VEGFA and PLGF or VEGFA, VEGFR2 and PLGF, may be mathematically combined and the combined value may be correlated to the underlying diagnostic question. Marker values may be combined by any appropriate state of the art mathematical method. Well-known mathematical methods for correlating a marker combination to a disease or to a treatment effect employ methods like, discriminant analysis (DA) (i.e. linear-, quadratic-, regularized-DA), Kernel Methods (i.e. SVM), Nonparametric Methods (i.e. k-Nearest-Neighbor Classifiers), PLS (Partial Least Squares), Tree-Based Methods (i.e. Logic Regression, CART, Random Forest Methods, Boosting/Bagging Methods), Generalized Linear Models (i.e. Logistic Regression), Principal Components based Methods (i.e. SIMCA), Generalized Additive Models, Fuzzy Logic based Methods, Neural Networks and Genetic Algorithms based Methods. The skilled artisan will have no problem selecting an appropriate method to evaluate a marker combination of the present invention. The method used in correlating marker combinations in accordance with the invention herein disclosed with, for example improved overall survival, progression free survival, responsiveness or sensitivity to addition of bevacizumab to chemotherapeutic agents/chemotherapy regimen and/or the prediction of a response to or sensitivity to bevacizumab (in addition to one or more chemotherapeutic agents/chemotherapy regimen) is selected from DA (i.e. Linear-, Quadratic-, Regularized Discriminant Analysis), Kernel Methods (i.e. SVM), Nonparametric Methods (i.e. k-Nearest-Neighbor Classifiers), PLS (Partial Least Squares), Tree-Based Methods (i.e. Logic Regression, CART, Random Forest Methods, Boosting Methods), or Generalized Linear Models (i.e. Logistic Regression). Details relating to these statistical methods are found in the following references: Ruczinski, I., et al, J. of Computational and Graphical Statistics, 12 (2003) 475-511; Friedman, J. H., J. of the American Statistical Association 84 (1989) 165-175; Hastie, Trevor, Tibshirani, Robert, Friedman, Jerome, The Elements of Statistical Learning, Springer Series in Statistics, 2001; Breiman, L., Friedman, J. H., Olshen, R. A., Stone, C. J. (1984) Classification and regression trees, California: Wadsworth; Breiman, L., Random Forests, Machine Learning, 45 (2001) 5-32; Pepe, M. S., The Statistical Evaluation of Medical Tests for Classification and Prediction, Oxford Statistical Science Series, 28 (2003); and Duda, R. O., Hart, P. E., Stork, D. G., Pattern Classification, Wiley Interscience, 2nd Edition (2001).

Accordingly, the invention herein disclosed relates to the use of an optimized multivariate cut-off for the underlying combination of biological markers and to discriminate state A from state B, e.g. patients responsive to or sensitive to the addition of bevacizumab to a chemotherapy regimen from patients that are poor responders to the addition of bevacizumab therapy to a chemotherapy regimen. In this type of analysis the markers are no longer independent but form a marker panel or a combined expression level.

The present invention, therefore, relates to a method for improving the treatment effect of a chemotherapy regimen of patients suffering from breast cancer, in particular locally advanced, recurrent or metastatic HER-2 negative breast cancer, by adding bevacizumab to a chemotherapy regimen by determining the expression levels two or more of VEGFA, PLGF and VEGFR2, by adding these expression levels such that each expression level is multiplied with a weight function (or weighting factor). Surprisingly, the result ("value", result of the mathematical operation, or combined expression level) correlates with treatment effect in patients administered bevacizumab in combination chemotherapy regimens such that values above a pre-specified (multivariate) cut-off are indicative of better treatment effect for the patient and values below this cut-off are indicative of poorer treatment effect.

Accordingly, the invention relates to a method for improving the treatment effect of a chemotherapy regimen of patients suffering from breast cancer, in particular locally advanced, recurrent or metastatic HER-2 negative breast cancer, by adding bevacizumab to a chemotherapy regimen by determining the expression levels two or more of VEGFA and VEGFR2, by adding these expression levels such that each expression level is multiplied with a weight function (or weighting factor). Surprisingly, the result ("value", result of the mathematical operation, or combined expression level) correlates with treatment effect in patients administered bevacizumab in combination chemotherapy regimens such that values above a pre-specified (multivariate) cut-off are indicative of better treatment effect for the patient and values below this cut-off are indicative of poorer treatment effect.

The invention relates to a method for improving the treatment effect of a chemotherapy regimen of patients suffering from breast cancer, in particular locally advanced, recurrent or metastatic HER-2 negative breast cancer, by adding bevacizumab to a chemotherapy regimen by determining the expression levels two or more of VEGFA and PLGF, by adding these expression levels such that each expression level is multiplied with a weight function (or weighting factor). Surprisingly, the result ("value", result of the mathematical operation, or combined expression level) correlates with treatment effect in patients administered bevacizumab in combination chemotherapy regimens such that values above a pre-specified (multivariate) cut-off are indicative of better treatment effect for the patient and values below this cut-off are indicative of poorer treatment effect.

The present invention, accordingly, relates to a method for improving the treatment effect of a chemotherapy regimen of patients suffering from breast cancer, in particular locally advanced, recurrent or metastatic HER-2 negative breast cancer, by adding bevacizumab to a chemotherapy regimen by determining the expression levels of VEGFA, VEGFR2 and PLGF, and by adding these expression levels such that each expression level is multiplied with a weight function (or weighting factor). Surprisingly, the result ("value", result of the mathematical operation, or combined expression level) correlates with treatment effect in patients administered bevacizumab in combination chemotherapy regimens such that values above a pre-specified (multivariate) cut-off are indicative of better treatment effect for the patient and values below this cut-off are indicative of poorer treatment effect.

For example, as shown in the appended illustrative example, the following equations can be used for assessing the combined expression level of VEGFA and VEGFR2 or VEGFA and PLGF when the treatment effect is progression free survival in patients suffering from locally advanced, recurrent or metastatic HER-2 negative breast cancer and the treatment is bevacizumab at low (7.5 mg/kg every three weeks) or high dose (15 mg/kg every three weeks) plus docetaxel therapy in comparison to placebo plus docetaxel therapy.

Formula 1: norm(VEGFA)+1.3*norm(VEGFR2)
Equivalent formula: 0.71*log2(VEGFA)+3.16*log2(VEGFR2)-15.6
and
Formula 2: 0.25*norm(VEGFA)+0.21*norm(PLGF)
Equivalent formula: 0.18*log2(VEGFA)+0.42*log2(PLGF)-3.1

Where we use log2 transformation and

$$x_i \rightarrow norm(x_i) = \frac{\log 2(x_i) - median(\log 2(x))}{mad(\log 2(x))}$$

Where mad is the median absolute deviation adjusted by a factor of 1.4826.

Accordingly, in the context of the herein described invention, and in accordance with the appended illustrative example, a high combined expression level of VEGFA and VEGFR2 is Formula 1 $\geq$ -0.132 and a low combined expression of VEGFA and VEGFR2 is Formula 1 < -0.132, with regard to progression free survival for the addition of both low and high dose bevacizumab therapy. In the context of the herein described invention, and in accordance with the appended illustrative example, a high combined expression level of VEGFA and PLGF is Formula 2 $\geq$ -0.006 and a low combined expression of VEGFA and PLGF is Formula 2 < -0.006, with regard to progression free survival for the addition of both low and high dose bevacizumab therapy. The skilled person will, however, understand that the expression levels measured for markers of a marker panel (or a combined expression level), e.g. for VEGFA and VEGFR2 or VEGFA and PLGF, may be mathematically combined and the combined expression level may be correlated to the underlying diagnostic question in more than one way. Accordingly, marker levels may be combined by any appropriate state of the art mathematical method.

The expression level of one or more of the markers VEGFA, VEGFR2 and PLGF may be assessed by any method known in the art suitable for determination of specific protein levels in a patient sample and is preferably determined by an immunoassay method, such as ELISA, employing antibodies specific for one or more of VEGFA, VEGFR2 and PLGF. Such methods are well known and routinely implemented in the art and corresponding commercial antibodies and/or kits are readily available. For example, commercially available antibodies/test kits for VEGFA, VEGFR2 and PLGF can be obtained from Bender RELIATech and R&D Systems as clone 3C5 and 26503 , from R&D systems as clone 89115 and 89109 and from Roche Diagnostics GmbH as clone 2D6D5 and 6A11D2, respectively. Preferably, the expression levels of the marker/indicator proteins of the invention are assessed using the reagents and/or protocol recommendations of the antibody or kit manufacturer. The skilled person will also be aware of further means for determining the expression level of one or more of VEGFA, VEGFR2 and PLGF by immunoassay methods. Therefore, the expression level of one or more of the markers/indicators of the invention can be routinely and reproducibly determined by a person skilled in the art without undue burden. However, to ensure accurate and reproducible results, the invention also encompasses the testing of patient samples in a specialized laboratory that can ensure the validation of testing procedures.

$VEGF_{12}$, and $VEGF_{110}$ protein can be detected using any method known in the art. For example, tissue or cell samples from mammals can be conveniently assayed for, *e.g.,* proteins using Westerns, ELISAs, etc.Many references are available to provide guidance in applying the above techniques (Kohler et al., Hybridoma Techniques (Cold Spring Harbor Laboratory, New York, 1980); Tijssen, Practice and Theory of Enzyme Immunoassays (Elsevier, Amsterdam, 1985); Campbell, Monoclonal Antibody Technology (Elsevier, Amsterdam, 1984); Hurrell, Monoclonal Hybridoma Antibodies: Techniques and Applications (CRC Press, Boca Raton, FL, 1982); and Zola, Monoclonal Antibodies: A Manual of Techniques, pp. 147-1 58 (CRC Press, Inc., 1987)).

If reference is made to the detection or level of $VEGF_{121}$ and $VEGF_{110}$ this means that the sum of both molecules is measured, e.g., using an assay that detects both $VEGF_{121}$ and $VEGF_{110}$. Assays that detect both molecules $VEGF_{121}$ and $VEGF_{110}$ include, e.g., assays that have a sensitivity for the corresponding other form, (i.e. for $VEGF_{121}$ if $VEGF_{110}$ is better recognized, or for $VEGF_{110}$ if $VEGF_{121}$ is better recognized, respectively) of at least 25%. In certain embodiments, in the assays have sensitivity to the corresponding other form of at least 50%, 75%, 80%, 85%, 90% or above. In one embodiment both $VEGF_{121}$ and $VEGF_{110}$ are measured with essentially the same sensitivity.

As to detection of $VEGF_{121}$ and $VEGF_{110}$ protein, various assays are available. For example, the sample may be contacted with an antibody or an antibody combination (e.g. in a sandwich assay) preferentially or specifically binding the short VEGF-A isoforms, $VEGF_{121}$ and $VEGF_{110}$, respectively as compared to the longer naturally occurring VEGF-A isoforms $VEGF_{165}$ and $VEGF_{189}$, respectively. Preferably the short isoforms are detected with an at least 3-fold higher sensitivity as compared to the longer isoforms. An at least 3-fold higher sensitivity is acknowledged if a standard curve is established using a short isoform (purity at least 90% by SDS-PAGE and concentration determined by OD 280nm) and for a long isoform at a predetermined concentration (purity at least 90% by SDS-PAGE and concentration determined by OD 280nm) using the same reagents and the same standard curve the value read of the standard curves is only one third or less of the expected concentration. Also preferred the sensitivity for the short isoforms is at least 4-fold, 5-fold, 6-fold, 7-fold, 8-fold or 9-fold higher as compared to the long isoforms, especially as compared to $VEGF_{165}$.

In one embodiment both short isoforms $VEGF_{121}$ and $VEGF_{110}$ are specifically detected. Such specific detection is e.g. possible if antibodies, especially monoclonal antibodies are used and employed that bind to the sequence generated by joining exons 4 and 8 in $VEGF_{121}$ or the free C-terminal end of $VEGF_{110}$, respectively. Such $VEGF_{110}$ anti C-terminus antibody does not bind to any VEGF-A isoform comprising amino acid 110 as part of a longer polypeptide chain or to shorter VEGF-A fragments ending e.g. at amino acid 109. The monoclonal antibody that binds to the sequence generated by joining exons 4 and 8, respectively, in $VEGF_{121}$ will not bind to the amino acid sequences comprised in the longer VEGF isoforms 165 and 189, respectively, since therein other amino acid sequences are present due to the joining of exon 4 and exon 7, and of exon 4 and exon 5, respectively (see: Ferrara, N., Mol. Biol. of the Cell 21 (2010) 687-690). Specific binding in the above sense is acknowledged, if the antibody used exhibits less than 10% cross-reactivity with a shorter fragment and less than 10% cross-reactivity with those VEGF-A isoforms not having a free C-terminal amino 110 in case of the anti- $VEGF_{110}$ antibody, or those isoforms not comprising the sequence generated by joining exons 4 and 8 in case of the anti-$VEGF_{121}$ antibody, respectively. Also preferred the cross-reactivity will be less than 5%, 4%, 3%, 2% and 1%, respectively, for both shorter fragments and not having a free C-terminal amino acid 110 or VEGF isoforms not having the sequence generated by joining exons 4 and 8, respectively.

Appropriate specific antibodies only binding the short VEGF isoforms $VEGF_{121}$ or $VEGF_{110}$, respectively, can be obtained according to standard procedures. Usually a peptide representing or comprising the C-terminal most at least 4, 5, 6, 7, 8, 9, 10 or more amino acids of $VEGF_{110}$ or a peptide representing or comprising at least 5, 6, 7, 8, 9, 10 or more amino acids comprising amino acids C-terminal and N-terminal to amino acid 115 of $VEGF_{121}$, respectively, will be synthesized, optionally coupled to a carrier and used for immunization. Specific polyclonal antibodies can be obtained by appropriate immunosorption steps. Monoclonal antibodies can easily be screened for reactivity with $VEGF_{121}$ or $VEGF_{110}$, respectively, and appropriate low cross-reactivity. Low cross-reactivity in terms of the VEGF110-specific antibody can be assessed for both shorter fragments of $VEGF_{110}$ (e.g. lacking the C-terminal amino acid of $VEGF_{110}$) and VEGF-A isoforms not having a free C-terminal amino acid of $VEGF_{110}$. Low cross-reactivity in terms of the $VEGF_{121}$-specific antibody can be assessed using VEGF-isoforms containing the amino acid sequences formed upon joining of exon 4 and exon 7, and of exon 4 and exon 5, respectively.

$VEGF_{111}$ protein or nucleic acids can be detected using any method known in the art. For example, tissue or cell samples from mammals can be conveniently assayed for, *e.g.,* proteins using Westerns, ELISAs, mRNAs or DNAs from a genetic biomarker of interest using Northern, dot-blot, or polymerase chain reaction (PCR) analysis, array hybridization, RNase protection assay, or using DNA SNP chip microarrays, which are commercially available, including DNA microarray snapshots. For example, real-time PCR (RT-PCR) assays such as quantitative PCR assays are well known in the art. In an illustrative embodiment of the invention, a method for detecting mRNA from a genetic biomarker of interest in a biological sample comprises producing cDNA from the sample by reverse transcription using at least one primer; amplifying the cDNA so produced; and detecting the presence of the amplified cDNA. In addition, such methods can include one or more steps that allow one to determine the levels of mRNA in a biological sample (e.g., by simultaneously examining the levels a comparative control mRNA sequence of a "housekeeping" gene such as an actin family member). Optionally, the sequence of the amplified cDNA can be determined.

Many references are available to provide guidance in applying the above techniques (Kohler et al., Hybridoma Techniques (Cold Spring Harbor Laboratory, New York, 1980); Tijssen, Practice and Theory of Enzyme Immunoassays (Elsevier, Amsterdam, 1985); Campbell, Monoclonal Antibody Technology (Elsevier, Amsterdam, 1984); Hurrell, Monoclonal Hybridoma Antibodies: Techniques and Applications (CRC Press, Boca Raton, FL, 1982); and Zola, Monoclonal Antibodies: A Manual of Techniques, pp. 147-1 58 (CRC Press, Inc., 1987).

As to detection of $VEGF_{111}$ protein, various assays are available For example, the sample may be contacted with an antibody or an antibody combination (e.g. in a sandwich assay) preferentially or specifically binding to $VEGF_{111}$ as compared to the longer naturally occurring VEGF-A isoforms $VEGF_{165}$ and $VEGF_{189}$, respectively. Preferably the short isoform $VEGF_{111}$ is detected with an at least 3-fold higher sensitivity as compared to the longer isoforms. An at least 3-fold higher sensitivity is acknowledged if a standard curve is established using a short isoform (purity at least 90% by SDS-PAGE and concentration determined by OD 280nm) and for a long isoform at a predetermined concentration (purity at least 90% by SDS-PAGE and concentration determined by OD 280nm) using the same reagents and the same standard curve the value read of the standard curves is only one third or less of the expected concentration. Also preferred the sensitivity for the short isoforms is at least 4-fold, 5-fold, 6-fold, 7-fold, 8-fold or 9-fold higher as compared to the long isoforms.

In one embodiment isoform $VEGF_{111}$ is specifically detected. Such specific detection is e.g. possible if antibodies, especially monoclonal antibodies are used and employed that bind to the exon junction unique for $VEGF_{111}$. Such antibody does not bind to other VEGF-A isoform or cleavage products thereof not comprising this specific exon junction. Specific binding in the above sense is acknowledged, if the antibody used exhibits less than 10% cross-reactivity with other VEGF-A isoforms, like $VEGF_{121}$ or $VEGF_{165}$, respectively, not having this unique exon junction. Also preferred the cross-reactivity to e.g. $VEGF_{121}$ will be less than 5%, 4%, 3%, 2% and 1%, respectively.

Specificity for $VEGF_{111}$ in one embodiment is assessed by comparing VEGF111 (purity at least 90% by SDS-PAGE and concentration determined by OD 280nm) and VEGF121 (purity at least 90% by SDS-PAGE and concentration determined by OD 280nm) using the same reagents. If in this comparison the signal obtained for $VEGF_{121}$ material is only one tens or less of the signal as obtained with the $VEGF_{111}$ material, then cross-reactivity towards $VEGF_{121}$ is less than 10%. As the skilled artisan will appreciate the $VEGF_{121}$ signal is preferably read of at a concentration which yields about 50% of the maximal signal for $VEGF_{111}$.

Appropriate specific antibodies only binding the short VEGF isoform $VEGF_{111}$ can be obtained according to standard procedures. Usually a peptide representing or comprising amino acids C-terminal and N-terminal to amino acid 105 of $VEGF_{111}$ will be synthesized, optionally coupled to a carrier and used for immunization. Preferably such peptide will be at least six amino acids long and comprise at least the amino acids 105 and106 of VEGF$_{111}$. Also preferred it will comprise at least the amino acids 104, 105, 106 and 107 of VEGF$_{111}$. As the skilled artisan will appreciate longer peptides comprising e.g. 3 or more amino acids N- and C-terminal to the exon junction between amino acids 105 and 106 of VEGF$_{111}$ can also be used to obtain antibodies specifically binding VEGF$_{111}$.

Unmodified VEGF protein can be detected using any appropriate method known in the art. Preferably an antibody will be used having at least the preferential binding properties to unmodified VEGF as compared to modified VEGF as MAB 3C5, which is commercially available from RELIATech GmbH, Wolfenbüttel, Germany. For example, tissue or cell samples from mammals can be conveniently assayed for the unmodified VEGF protein using Westerns, ELISAs, etc. Many references are available to provide guidance in applying the above techniques (Kohler et al., Hybridoma Techniques (Cold Spring Harbor Laboratory, New York, 1980); Tijssen, Practice and Theory of Enzyme Immunoassays (Elsevier, Amsterdam, 1985); Campbell, Monoclonal Antibody Technology (Elsevier, Amsterdam, 1984); Hurrell, Monoclonal Hybridoma Antibodies: Techniques and Applications (CRC Press, Boca Raton, FL, 1982); and Zola, Monoclonal Antibodies: A Manual of Techniques, pp. 147-1 58 (CRC Press, Inc., 1987)).

If reference is made to the detection or level of unmodified VEGF this means that unmodified VEGF-molecules (isoforms or cleavage products) as e.g. bound by MAB 3C5 are measured.

As to detection of unmodified VEGF protein, various assays are available. For example, the sample may be contacted with an antibody or an antibody combination (e.g. in a sandwich assay) preferentially or specifically binding to unmodified VEGF as compared to modified VEGF, e.g. as naturally occurring in a patient's sample. Preferably unmodified VEGF is detected using an antibody specifically binding to unmodified VEGF, i.e., with an antibody having at least 3-fold higher sensitivity for unmodified VEGF165 as compared to modified VEGF165. Such at least 3-fold higher sensitivity for unmodified VEGF is assessed by comparing VEGF165 recombinantly produced in E. coli (purity at least 90% by SDS-PAGE and concentration determined by OD 280nm) and VEGF165 recombinantly produced in HEK cells (purity at least 90% by SDS-PAGE and concentration determined by OD 280nm) using the same reagents. If in this comparison the signal obtained for the HEK-produced material is only one third or less of the signal as obtained with the E. coli-derived material, then unmodified VEGF is detected with an at least 3-fold higher sensitivity. As the skilled artisan will appreciate the signal is preferably read of at about 50% of the maximal signal. Preferably in this assessment the assay of example 5 is used. Also preferred the antibody specifically binding to unmodified VEGF (VEGF165 ex E. coli) is an antibody that detects unmodified VEGF with and at least 4-fold, 5-fold, 6-fold, 7-fold, 8-fold, 9-fold or 10-fold higher sensitivity as compared to the modified VEGF material (VEGF165 ex HEK cells).

In one embodiment unmodified VEGF is specifically detected using an antibody having at least the same binding preference for unmodified VEGF as compared to modified VEGF as the commercially available MAB 3C5. In one embodiment the relative sensitivity for or preferential binding of an antibody to unmodified VEGF is assessed in a sandwich immuno assay, wherein the antibody to unmodified VEGF is used as a capture antibody and a detection antibody is used that binds to an epitope present on all major VEGF-isoforms or cleavage products. In one embodiment the detection antibody will bind to an epitope outside the epitope for MAB 3C5, i.e., it will not bind to an epitope comprised in a synthetic peptide spanning amino acids 33 to 43 of VEGF. Preferably the detection antibody will bind to an epitope comprised in the amino acids ranging from 1 to 32, form 44 to 105, to the last six amino acids of mature VEGF165, or to a conformational epitope not overlapping with the epitope bound by MAB 3C5. In one embodiment the antibody specifically binding unmodified VEGF165 as compared to modified VEGF has the property to bind to an epitope comprised in a synthetic peptide spanning amino acids 33 to 43 of VEGF.

Appropriate specific antibodies specifically binding unmodified VEGF can be obtained according to standard procedures. Usually an isoform of VEGF produced recombinantly in E. coli or obtained synthetically e.g. by solid phase polypeptide synthesis, or a peptide representing or comprising an epitope of VEGF produced recombinantly in E. coli or obtained synthetically e.g. by solid phase polypeptide synthesis will be used as an immunogen. Monoclonal antibodies can easily be produced according to standard protocols and screened for reactivity with unmodified VEGF and appropriate low cross-reactivity with modified VEGF. One convenient and preferred screening method is based on the use of VEGF165 recombinantly produced in E. coli (purity at least 90% by SDS-PAGE and concentration determined by OD 280nm) and of VEGF165 recombinantly produced in HEKcells (purity at least 90% by SDS-PAGE and concentration determined by OD 280nm), respectively.

The expression level of one or more of VEGFA, VEGFR2 and PLGF may be assessed in a patient sample that is a biological sample. The patient sample may be a blood sample, blood serum sample or a blood plasma sample. In one embodiment, the sample is EDTA-plasma. In one embodiment, the sample is citrate-plasma. Methods of obtaining blood samples, blood serum samples and blood plasma samples are well known in the art. The patient sample may be obtained from the patient prior to or after neoadjuvant therapy or prior to or after adjuvant therapy.

In the context of the present invention, bevacizumab is to be administered in addition to or as a co-therapy or co-treatment with one or more chemotherapeutic agents administered as part of standard chemotherapy regimen as known in the art. Examples of agents included in such standard chemotherapy regimens include 5-fluorouracil, leucovorin, irinotecan, gemcitabine, erlotinib, capecitabine, taxanes, such as docetaxel and paclitaxel, interferon alpha, vinorelbine, and platinum-based chemotherapeutic agents, such as, carboplatin, cisplatin and oxaliplatin. As demonstrated in the appended illustrative example, the addition of bevacizumab to effected an increase in the progression free survival in the patients and/or patient population defined and selected according to the expression level of one or more of VEGFA, VEGFR2 and PLGF. Thus, bevacizumab may be combined with a chemotherapy regimen, such as docetaxel therapy as demonstrated in the appended illustrative example.

Common modes of administration include parenteral administration as a bolus dose or as an infusion over a set period of time, e.g., administration of the total daily dose over 10 min., 20 min., 30 min., 40 min., 50 min., 60 min., 75 min., 90 min., 105 min., 120 min., 3 hr., 4 hr., 5 hr. or 6 hr. For example, 2.5 mg/kg of body weight to 15 mg/kg of body weight bevacizumab (Avastin®) can be administered every week, every 2 weeks or every 3 weeks, depending on the type of cancer being treated. Examples of dosages include 2.5 mg/kg of body weight, 5 mg/kg of body weight, 7.5 mg/kg of body weight, 10 mg/kg of body weight and 15 mg/kg of body weight given every week, every 2 weeks or every 3 weeks. Further examples of dosages are 5 mg/kg of body weight every 2 weeks, 10 mg/kg every 2 weeks, 7.5 mg/kg of body weight every 3 weeks and 15 mg/kg of body weight every 3 weeks. In the context of the herein described invention, low dose bevacizumab includes, for example, dosages of 2.5 mg/kg of body weight every week, 5 mg/kg of body weight every 2 weeks and 7.5 mg/kg of body weight every 3 weeks. In the context of the herein described invention, high dose bevacizumab includes, for example, dosages of 5 mg/kg of body weight every week, 10 mg/kg of body weight every 2 weeks and 15 mg/kg of body weight every 3 weeks. For the treatment of breast cancer, in particular locally advanced, recurrent or metastatic HER-2 negative breast cancer, dosages include low dose bevacizumab, in particular 7.5 mg/kg every 3 weeks, and high dose bevacizumab, in particular 15 mg/kg of body weight given once every 3 weeks. The skilled person will recognize that further modes of administration of bevacizumab are encompassed by the invention as determined by the specific patient and chemotherapy regimen, and that the specific mode of administration and therapeutic dosage are best determined by the treating physician according to methods known in the art.

The patients selected according to the methods of the present invention are treated with bevacizumab in combination with a chemotherapy regimen, and may be further treated with one or more additional anti-cancer therapies. In certain aspects, the one or more additional anti-cancer therapy is radiation.

The present invention also relates to a diagnostic composition or kit comprising oligonucleotides or polypeptides suitable for the determination of expression levels of one or more of VEGFA, VEGFR2 and PLGF. As detailed herein, oligonucleotides such as DNA, RNA or mixtures of DNA and RNA probes may be of use in detecting mRNA levels of the marker/indicator proteins, while polypeptides may be of use in directly detecting protein levels of the marker/indicator proteins via specific protein-protein interaction. In preferred aspects of the invention, the polypeptides encompassed as probes for the expression levels of one or more of VEGFA, VEGFR2 and PLGF, and included in the kits or diagnostic compositions described herein, are antibodies specific for these proteins, or specific for homologues and/or truncations thereof.

Accordingly, in a further embodiment of the present invention provides a kit useful for carrying out the methods herein described, comprising oligonucleotides or polypeptides capable of determining the expression level of one or more of VEGFA, VEGFR2 and PLGF. The oligonucleotides may comprise primers and/or probes specific for the mRNA encoding one or more of the markers/indicators described herein, and the polypeptides comprise proteins capable of specific interaction with the marker/indicator proteins, e.g., marker/indicator specific antibodies or antibody fragments.

Accordingly, the present invention relates to bevacizumab for use in an improved chemotherapy regimen for a patient suffering from breast cancer wherein the expression level of one or more of VEGFA, VEGFR2 and PLGF in a patient sample is determined and whereby a patient having an increased level of one or more of VEGFA, VEGFR2 and PLGF relative to control levels determined in patients diagnosed with breast cancer is administered bevacizumab in addition to the chemotherapy regimen.

The following similar uses can be applied mutatis mutandis.

The present invention relates to the use of bevacizumab for improving the treatment effect of a chemotherapy regimen of a patient suffering from breast cancer comprising the following steps:

(a) determining the expression level of one or more of VEGFA, VEGFR2 and PLGF in a patient sample; and
(b) administering bevacizumab in combination with the chemotherapy regimen to the patient having an increased level of one or more of VEGFA, VEGFR2 and PLGF relative to control levels determined in patients diagnosed with breast cancer.

The breast cancer may be locally advanced, recurrent or metastatic HER-2 negative breast cancer. The chemotherapy regimen may comprise taxane therapy, such as docetaxel or paclitaxel therapy.

Accordingly, the present invention relates to the use of bevacizumab for improving the treatment effect of a chemotherapy regimen of a patient suffering from breast cancer comprising the following steps:

(a) obtaining a sample from said patient;

(b) determining the expression level of one or more of VEGFA, VEGFR2 and PLGF; and
(c) administering bevacizumab in combination with the chemotherapy regimen to the patient having an increased level of one or more of VEGFA, VEGFR2 and PLGF relative to control levels determined in patients diagnosed breast cancer.

The breast cancer may be locally advanced, recurrent or metastatic HER-2 negative breast cancer. The chemotherapy regimen may comprise taxane therapy, such as docetaxel or paclitaxel therapy.

The present invention relates to the use of bevacizumab for improving the progression free survival of a patient suffering from breast cancer comprising the following steps:

(a) determining the expression level of one or more of VEGFA, VEGFR2 and PLGF in a patient sample; and
(b) administering bevacizumab in combination with the chemotherapy regimen to the patient having an increased level of one or more of VEGFA VEGFR2 and PLGF relative to control levels determined in patients diagnosed with breast cancer.

The breast cancer may be locally advanced, recurrent or metastatic HER-2 negative breast cancer. The chemotherapy regimen may comprise taxane therapy, such as docetaxel or paclitaxel therapy.

Accordingly, the present invention relates to the use of bevacizumab for improving the progression free survival of a patient suffering from breast cancer comprising the following steps:

(a) obtaining a sample from said patient;
(b) determining the expression level of one or more of VEGFA, VEGFR2 and PLGF; and
(c) administering bevacizumab in combination with the chemotherapy regimen to the patient having an increased level of one or more of VEGFA, VEGFR2 and PLGF relative to control levels determined in patients diagnosed breast cancer.

The breast cancer may be locally advanced, recurrent or metastatic HER-2 negative breast cancer. The chemotherapy regimen may comprise taxane therapy, such as docetaxel or paclitaxel therapy.

The present invention provides the use of bevacizumab for improving progression free survival of a patient suffering from breast cancer comprising the following steps:

(a) determining the expression level of VEGFA or VEGFR2 in a patient sample; and
(b) administering bevacizumab in combination with a chemotherapy regimen to the patient having an increased combined expression level of VEGFA or VEGFR2 relative to a control combined expression level determined in patients diagnosed with breast cancer.

The breast cancer may be locally advanced, recurrent or metastatic HER-2 negative breast cancer. The chemotherapy regimen may comprise taxane therapy, such as docetaxel or paclitaxel therapy. The dose of bevacizumab administered may be low or high dose bevacizumab.

The present invention relates to the use of bevacizumab for improving progression free survival of a patient suffering from breast cancer comprising the following steps:

(a) obtaining a sample from said patient;
(b) determining the expression level of VEGFA or VEGFR2; and
(c) administering bevacizumab in combination with a chemotherapy regimen to the patient having an increased combined expression level of VEGFA or VEGFR2 relative to a control combined expression level determined in patients diagnosed with breast cancer.

The breast cancer may be locally advanced, recurrent or metastatic HER-2 negative breast cancer. The chemotherapy regimen may comprise taxane therapy, such as docetaxel or paclitaxel therapy. The dose of bevacizumab administered may be low or high dose bevacizumab.

The present invention provides the use of bevacizumab for improving progression free survival of a patient suffering from locally advanced, recurrent or metastatic HER-2 negative breast cancer comprising the following steps:

(a) determining the expression level of VEGFA or VEGFR2 in a patient sample; and
(b) administering bevacizumab in combination with a chemotherapy regimen to the patient having an increased combined expression level of VEGFA or VEGFR2 relative to a control combined expression level determined in patients diagnosed with said breast cancer.

The chemotherapy regimen may comprise taxane therapy, such as docetaxel or paclitaxel therapy. The dose of bevacizumab administered may be low or high dose bevacizumab.

The present invention relates to the use of bevacizumab for improving progression free survival of a patient suffering from locally advanced, recurrent or metastatic HER-2 negative breast cancer comprising the following steps:

(a) obtaining a sample from said patient;
(b) determining the expression level of VEGFA or VEGFR2; and
(c) administering bevacizumab in combination with a chemotherapy regimen to the patient having an increased combined expression level of VEGFA or VEGFR2 relative to a control combined expression level determined in patients diagnosed with said breast cancer.

The chemotherapy regimen may comprise taxane therapy, such as docetaxel or paclitaxel therapy. The dose of bevacizumab administered may be low or high dose bevacizumab.

The invention relates to the use of bevacizumab for improving progression free survival of a patient suffering from locally advanced, recurrent or metastatic HER-2 negative breast cancer comprising the following steps:

(a) determining the expression level of VEGFA or VEGFR2 in a patient sample; and
(b) administering bevacizumab in combination with a chemotherapy regimen to the patient having an increased combined expression level of VEGFA or VEGFR2 relative to a control combined expression level determined in patients diagnosed with said breast cancer,

wherein the chemotherapy regimen is docetaxel therapy. The dose of bevacizumab administered may be low or high dose bevacizumab.

Accordingly, the present invention relates to the use of bevacizumab for progression free survival of a patient suffering from locally advanced, recurrent or metastatic HER-2 negative breast cancer comprising the following steps:

(a) obtaining a sample from said patient;
(b) determining the expression level of VEGFA or VEGFR2; and
(c) administering bevacizumab in combination with a chemotherapy regimen to the patient having an increased combined expression level of VEGFA or VEGFR2 relative to a control combined expression level determined in patients diagnosed with said breast cancer,

wherein the chemotherapy regimen is docetaxel therapy. The dose of bevacizumab administered may be low or high dose bevacizumab.

The present invention provides the use of bevacizumab for improving progression free survival of a patient suffering from breast cancer comprising the following steps:

(a) determining the expression level of VEGFA and VEGFR2 in a patient sample; and
(b) administering bevacizumab in combination with a chemotherapy regimen to the patient having an increased combined expression level of VEGFA and VEGFR2 relative to a control combined expression level determined in patients diagnosed with breast cancer.

The breast cancer may be locally advanced, recurrent or metastatic HER-2 negative breast cancer. The chemotherapy regimen may comprise taxane therapy, such as docetaxel or paclitaxel therapy. The dose of bevacizumab administered may be low or high dose bevacizumab. As shown in the appended illustrative example, the combined expression level of VEGFA and VEGFR2 was particularly predictive in patients administered low dose bevacizumab.

The present invention relates to the use of bevacizumab for improving progression free survival of a patient suffering from breast cancer comprising the following steps:

(a) obtaining a sample from said patient;
(b) determining the expression level of VEGFA and VEGFR2; and
(c) administering bevacizumab in combination with a chemotherapy regimen to the patient having an increased combined expression level of VEGFA and VEGFR2 relative to a control combined expression level determined in patients diagnosed with breast cancer.

The breast cancer may be locally advanced, recurrent or metastatic HER-2 negative breast cancer. The chemotherapy regimen may comprise taxane therapy, such as docetaxel or paclitaxel therapy. The dose of bevacizumab administered may be low or high dose bevacizumab. As shown in the appended illustrative example, the combined expression level of VEGFA and VEGFR2 was particularly predictive in patients administered low dose bevacizumab.

The present invention provides the use of bevacizumab for improving progression free survival of a patient suffering from locally advanced, recurrent or metastatic HER-2 negative breast cancer comprising the following steps:

(a) determining the expression level of VEGFA and VEGFR2 in a patient sample; and
(b) administering bevacizumab in combination with a chemotherapy regimen to the patient having an increased combined expression level of VEGFA and VEGFR2 relative to a control combined expression level determined in patients diagnosed with said breast cancer.

The chemotherapy regimen may comprise taxane therapy, such as docetaxel or paclitaxel therapy. The dose of bevacizumab administered may be low or high dose bevacizumab. As shown in the appended illustrative example, the combined expression level of VEGFA and VEGFR2 was particularly predictive in patients administered low dose bevacizumab.

The present invention relates to the use of bevacizumab for improving progression free survival of a patient suffering from locally advanced, recurrent or metastatic HER-2 negative breast cancer comprising the following steps:

(a) obtaining a sample from said patient;
(b) determining the expression level of VEGFA and VEGFR2; and
(c) administering bevacizumab in combination with a chemotherapy regimen to the patient having an increased combined expression level of VEGFA and VEGFR2 relative to a control combined expression level determined in patients diagnosed with said breast cancer.

The chemotherapy regimen may comprise taxane therapy, such as docetaxel or paclitaxel therapy. The dose of bevacizumab administered may be low or high dose bevacizumab. As shown in the appended illustrative example, the combined expression level of VEGFA and VEGFR2 was particularly predictive in patients administered low dose bevacizumab.

The invention relates to the use of bevacizumab for improving progression free survival of a patient suffering from locally advanced, recurrent or metastatic HER-2 negative breast cancer comprising the following steps:

(a) determining the expression level of VEGFA and VEGFR2 in a patient sample; and
(b) administering bevacizumab in combination with a chemotherapy regimen to the patient having an increased combined expression level of VEGFA and VEGFR2 relative to a control combined expression level determined in patients diagnosed with said breast cancer,

wherein the chemotherapy regimen is docetaxel therapy. The dose of bevacizumab administered may be low or high dose bevacizumab. As shown in the appended illustrative example, the combined expression level of VEGFA and VEGFR2 was particularly predictive in patients administered low dose bevacizumab.

Accordingly, the present invention relates to the use of bevacizumab for progression free survival of a patient suffering from locally advanced, recurrent or metastatic HER-2 negative breast cancer comprising the following steps:

(a) obtaining a sample from said patient;
(b) determining the expression level of VEGFA and VEGFR2; and
(c) administering bevacizumab in combination with a chemotherapy regimen to the patient having an increased combined expression level of VEGFA and VEGFR2 relative to a control combined expression level determined in patients diagnosed with said breast cancer,

wherein the chemotherapy regimen is docetaxel therapy. The dose of bevacizumab administered may be low or high dose bevacizumab. As shown in the appended illustrative example, the combined expression level of VEGFA and VEGFR2 was particularly predictive in patients administered low dose bevacizumab.

The present invention provides the use of bevacizumab for improving progression free survival of a patient suffering from breast cancer comprising the following steps:

(a) determining the expression level of VEGFA and PLGF in a patient sample; and
(b) administering bevacizumab in combination with a chemotherapy regimen to the patient having an increased combined expression level of VEGFA and PLGF relative to a control combined expression level determined in patients diagnosed with breast cancer.

The breast cancer may be locally advanced, recurrent or metastatic HER-2 negative breast cancer. The chemotherapy regimen may comprise taxane therapy, such as docetaxel or paclitaxel therapy. The dose of bevacizumab administered may be low or high dose bevacizumab.

Accordingly, the present invention relates to the use of bevacizumab for progression free survival of a patient suffering from breast cancer comprising the following steps:

(a) obtaining a sample from said patient;
(b) determining the expression level of VEGFA and PLGF; and
(c) administering bevacizumab in combination with a chemotherapy regimen to the patient having an increased combined expression level of VEGFA and PLGF relative to a control combined expression level determined in patients diagnosed with breast cancer.

The breast cancer may be locally advanced, recurrent or metastatic HER-2 negative breast cancer. The chemotherapy regimen may comprise taxane therapy, such as docetaxel or paclitaxel therapy. The dose of bevacizumab administered may be low or high dose bevacizumab.

The present invention provides the use of bevacizumab for improving progression free survival of a patient suffering from locally advanced, recurrent or metastatic HER-2 negative breast cancer comprising the following steps:

(a) determining the expression level of VEGFA and PLGF in a patient sample; and
(b) administering bevacizumab in combination with a chemotherapy regimen to the patient having an increased combined expression level of VEGFA and PLGF relative to a control combined expression level determined in patients diagnosed with said breast cancer.

The chemotherapy regimen may comprise taxane therapy, such as docetaxel or paclitaxel therapy. The dose of bevacizumab administered may be low or high dose bevacizumab.

Accordingly, the present invention relates to the use of bevacizumab for progression free survival of a patient suffering from locally advanced, recurrent or metastatic HER-2 negative breast cancer comprising the following steps:

(a) obtaining a sample from said patient;
(b) determining the expression level of VEGFA and PLGF; and
(c) administering bevacizumab in combination with a chemotherapy regimen to the patient having an increased combined expression level of VEGFA and PLGF relative to a control combined expression level determined in patients diagnosed with said breast cancer.

The chemotherapy regimen may comprise taxane therapy, such as docetaxel or paclitaxel therapy. The dose of bevacizumab administered may be low or high dose bevacizumab.

The present invention provides the use of bevacizumab for improving progression free survival of a patient suffering from locally advanced, recurrent or metastatic HER-2 negative breast cancer comprising the following steps:

(a) determining the expression level of VEGFA and PLGF in a patient sample; and
(b) administering bevacizumab in combination with a chemotherapy regimen to the patient having an increased combined expression level of VEGFA and PLGF relative to a control combined expression level determined in patients diagnosed with said breast cancer,

wherein the chemotherapy regimen is docetaxel therapy.
The dose of bevacizumab administered may be low or high dose bevacizumab.

Accordingly, the present invention relates to the use of bevacizumab for progression free survival of a patient suffering from locally advanced, recurrent or metastatic HER-2 negative breast cancer comprising the following steps:

(a) obtaining a sample from said patient;
(b) determining the expression level of VEGFA and PLGF; and
(c) administering bevacizumab in combination with a chemotherapy regimen to the patient having an increased combined expression level of VEGFA and PLGF relative to a control combined expression level determined in patients diagnosed with said breast cancer

wherein the chemotherapy regimen is docetaxel therapy.
The dose of bevacizumab administered may be low or high dose bevacizumab.

The present invention provides the use of bevacizumab for improving progression free survival of a patient suffering from breast cancer comprising the following steps:

(a) determining the expression level of VEGFA, VEGFR2 and PLGF in a patient sample; and
(b) administering bevacizumab in combination with a chemotherapy regimen to the patient having an increased combined expression level of VEGFA, VEGFR2 and PLGF relative to a control combined expression level determined in patients diagnosed with breast cancer.

The breast cancer may be locally advanced, recurrent or metastatic HER-2 negative breast cancer. The chemotherapy regimen may comprise taxane therapy, such as docetaxel or paclitaxel therapy. The dose of bevacizumab administered may be low or high dose bevacizumab.

The present invention relates to the use of bevacizumab for improving progression free survival of a patient suffering from breast cancer comprising the following steps:

(a) obtaining a sample from said patient;
(b) determining the expression level of VEGFA, VEGFR2 and PLGF; and
(c) administering bevacizumab in combination with a chemotherapy regimen to the patient having an increased combined expression level of VEGFA, VEGFR2 and PLGF relative to a control combined expression level determined in patients diagnosed with breast cancer.

The breast cancer may be locally advanced, recurrent or metastatic HER-2 negative breast cancer. The chemotherapy regimen may comprise taxane therapy, such as docetaxel or paclitaxel therapy. The dose of bevacizumab administered may be low or high dose bevacizumab.

The present invention provides the use of bevacizumab for improving progression free survival of a patient suffering from locally advanced, recurrent or metastatic HER-2 negative breast cancer comprising the following steps:

(a) determining the expression level of VEGFA, VEGFR2 and PLGF in a patient sample; and
(b) administering bevacizumab in combination with a chemotherapy regimen to the patient having an increased combined expression level of VEGFA, VEGFR2 and PLGF relative to a control combined expression level determined in patients diagnosed with said breast cancer.

The chemotherapy regimen may comprise taxane therapy, such as docetaxel or paclitaxel therapy. The dose of bevacizumab administered may be low or high dose bevacizumab.

The present invention relates to the use of bevacizumab for improving progression free survival of a patient suffering from locally advanced, recurrent or metastatic HER-2 negative breast cancer comprising the following steps:

(a) obtaining a sample from said patient;
(b) determining the expression level of VEGFA, VEGFR2 and PLGF; and
(c) administering bevacizumab in combination with a chemotherapy regimen to the patient having an increased combined expression level of VEGFA, VEGFR2 and PLGF relative to a control combined expression level determined in patients diagnosed with said breast cancer.

The chemotherapy regimen may comprise taxane therapy, such as docetaxel or paclitaxel therapy. The dose of bevacizumab administered may be low or high dose bevacizumab.

The invention relates to the use of bevacizumab for improving progression free survival of a patient suffering from locally advanced, recurrent or metastatic HER-2 negative breast cancer comprising the following steps:

(a) determining the expression level of VEGFA, VEGFR2 and PLGF in a patient sample; and
(b) administering bevacizumab in combination with a chemotherapy regimen to the patient having an increased combined expression level of VEGFA, VEGFR2 and PLGF relative to a control combined expression level determined in patients diagnosed with said breast cancer,

wherein the chemotherapy regimen is docetaxel therapy.
The dose of bevacizumab administered may be low or high dose bevacizumab.

Accordingly, the present invention relates to the use of bevacizumab for progression free survival of a patient suffering from locally advanced, recurrent or metastatic HER-2 negative breast cancer comprising the following steps:

(a) obtaining a sample from said patient;
(b) determining the expression level of VEGFA, VEGFR2 and PLGF; and
(c) administering bevacizumab in combination with a chemotherapy regimen to the patient having an increased combined expression level of VEGFA, VEGFR2 and PLGF relative to a control combined expression level determined in patients diagnosed with said breast cancer,

wherein the chemotherapy regimen is docetaxel therapy.

The dose of bevacizumab administered may be low or high dose bevacizumab.

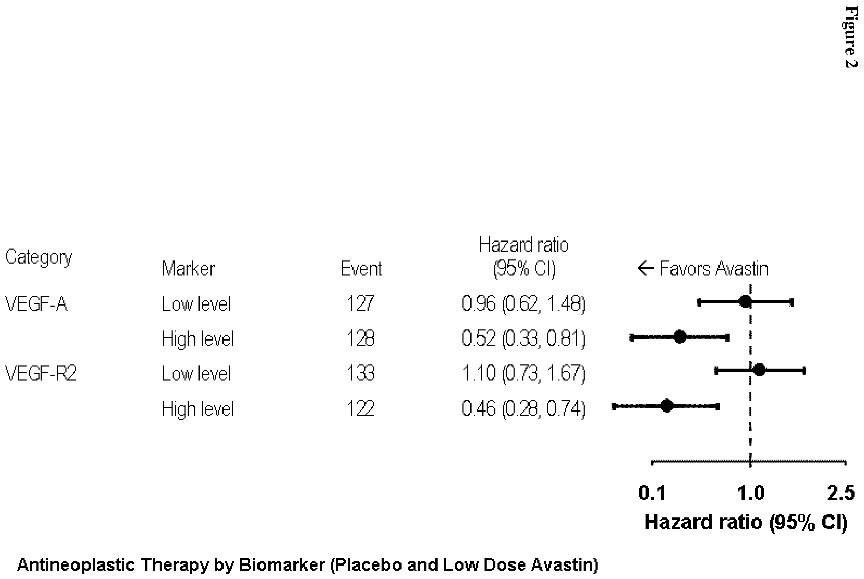

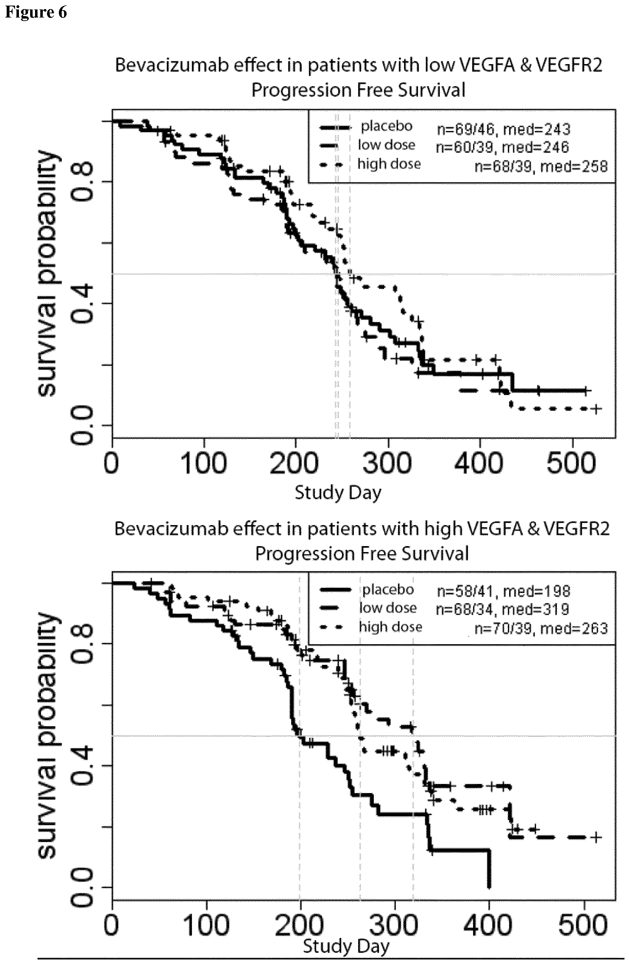

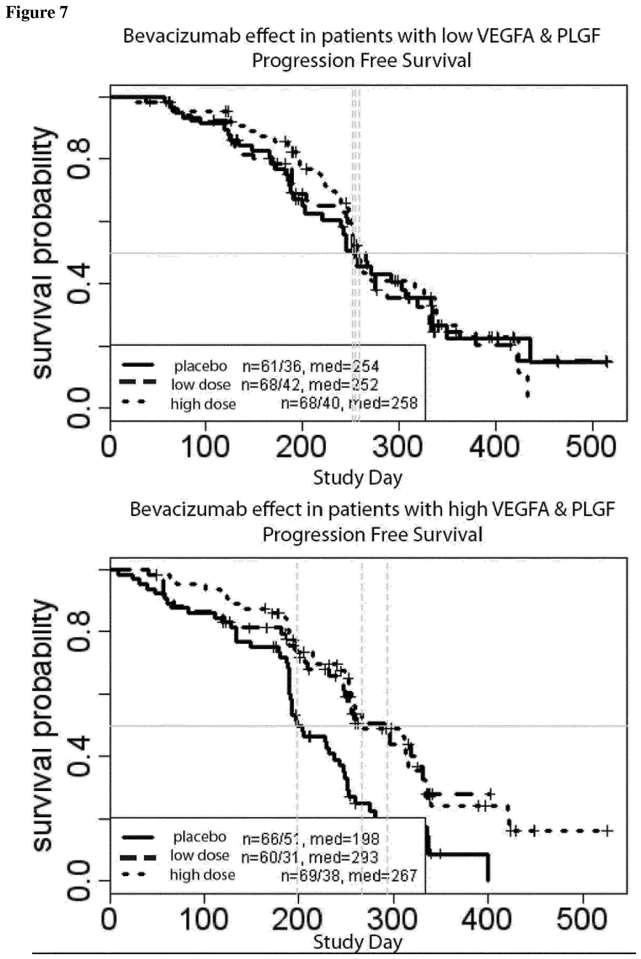

As documented in the appended illustrative example, the present invention solves the identified technical problem in that it could surprisingly be shown that the expression levels of one or more of VEGFA, VEGFR2 and PLGF in a given patient, relative to control levels determined in patients diagnosed with breast cancer, in particular locally advanced, recurrent or metastatic HER-2 negative breast cancer, correlate with treatment effect in patients administered bevacizumab in combination with a docetaxel chemotherapy regimen. As is shown in the appended illustrative example, it was surprisingly found that an increased protein expression level of VEGFA or VEGFR2 correlated with improved progression free survival of patients suffering from locally advanced, recurrent or metastatic HER-2 negative breast cancer that were treated with bevacizumab and a docetaxel chemotherapy regimen in comparison to patients treated with placebo and a docetaxel chemotherapy regimen (Figures 2 to 5). It was further surprisingly found that an increased combined expression level of VEGFA and VEGFR2 correlated with improved progression free survival of patients suffering from locally advanced, recurrent or metastatic HER-2 negative breast cancer that were treated with bevacizumab and a docetaxel chemotherapy regimen in comparison to patients treated with placebo and a docetaxel chemotherapy regimen (Figure 6). It was also surprisingly found that an increased combined expression level of VEGFA and PLGF correlated with progression free survival in patients suffering from locally advanced, recurrent or metastatic HER-2 negative breast cancer that were treated with bevacizumab and a docetaxel chemotherapy regimen in comparison to patients treated with placebo and a docetaxel chemotherapy regimen (Figure 7).

The invention, therefore, relates to an in vitro method of predicting the response to or sensitivity to the addition of bevacizumab to a chemotherapy regimen of a patient suffering from, suspect to suffer from or prone to suffer from breast cancer, in particular locally advanced, recurrent or metastatic HER-2 negative breast cancer, comprising determining the expression level, including combined expression levels, of one or more of VEGFA, VEGFR2 and PLGF in a patient sample. Accordingly, in the context of the methods described herein, the invention provides the use of specific probes, including for example binding molecules like antibodies and aptamers, for the preparation of a diagnostic composition for predicting the response to or sensitivity to the addition of bevacizumab to a chemotherapy regimen of a patient suffering from, suspect to suffer from or prone to suffer from breast cancer, in particular locally advanced, recurrent or metastatic HER-2 negative breast cancer, comprising determining the expression level, including combined expression levels, of one or more of VEGFA, VEGFR2 and PLGF in a patient sample. The invention, accordingly, relates to the use of specific probes, including for example binding molecules like antibodies and aptamers, for the preparation of a diagnostic composition for predicting the response to or sensitivity to the addition of bevacizumab to a chemotherapy regimen of a patient suffering from, suspect to suffer from or prone to suffer from breast cancer, in particular locally advanced, recurrent or metastatic HER-2 negative breast cancer, comprising determining the expression level of VEGFA or VEGFR2 in a patient sample.

The invention provides an in vitro method of predicting the response to or sensitivity to the addition of bevacizumab to a chemotherapy regimen of a patient suffering from, suspected to suffer from, or prone to suffer from breast cancer, in particular locally advanced, recurrent or metastatic HER-2 negative breast cancer, comprising determining the combined expression level of VEGFA and VEGFR2 in a patient sample. Accordingly, in the context of the methods described herein, the invention provides the use of specific probes, including for example binding molecules like antibodies and aptamers, for the preparation of a diagnostic composition for predicting the response to or sensitivity to the addition of bevacizumab to a chemotherapy regimen of a patient suffering from, suspect to suffer from or prone to suffer from breast cancer, in particular locally advanced, recurrent or metastatic HER2 negative breast cancer comprising determining the combined expression level of VEGFA and VEGFR2 in a patient sample.

The invention provides an in vitro method of predicting the response to or sensitivity to the addition of bevacizumab to a chemotherapy regimen of a patient suffering from, suspected to suffer from, or prone to suffer from breast cancer, in particular locally advanced, recurrent or metastatic HER2 negative breast cancer comprising determining the combined expression level of VEGFA and PLGF in a patient sample. Accordingly, in the context of the methods described herein, the invention provides the use of specific probes, including for example binding molecules like antibodies and aptamers, for the preparation of a diagnostic composition for predicting the response to or sensitivity to the addition of bevacizumab to a chemotherapy regimen of a patient suffering from, suspect to suffer from or prone to suffer from breast cancer, in particular locally advanced, recurrent or metastatic HER2 negative breast cancer comprising determining the combined expression level of VEGFA and PLGF in a patient sample.

The invention provides an in vitro method of predicting the response to or sensitivity to the addition of bevacizumab to a chemotherapy regimen of a patient suffering from, suspected to suffer from, or prone to suffer from breast cancer, in particular locally advanced, recurrent or metastatic HER-2 negative breast cancer comprising determining the combined expression level of VEGFA, VEGFR2 and PLGF in a patient sample. Accordingly, in the context of the methods described herein, the invention provides the use of specific probes, including for example binding molecules like antibodies and aptamers, for the preparation of a diagnostic composition for predicting the response to or sensitivity to the addition of bevacizumab to a chemotherapy regimen of a patient suffering from, suspect to suffer from or prone to suffer from breast cancer, in particular locally advanced, recurrent or metastatic HER-2 negative breast cancer comprising determining the combined expression level of VEGFA, VEGFR2 and PLGF in a patient sample.

The phrase "responsive to" in the context of the present invention indicates that a subject/patient suffering, suspected to suffer or prone to suffer breast cancer, in particular locally advanced, recurrent or metastatic HER-2 negative breast cancer, shows a response to a chemotherapy regimen comprising bevacizumab. A skilled person will readily be in a position to determine whether a person treated with bevacizumab according to the methods of the invention shows a response. For example, a response may be reflected by decreased suffering from the breast cancer, in particular locally advanced, recurrent or metastatic HER-2 negative breast cancer, such as a diminished and/or halted tumor growth, reduction of the size of a tumor, and/or amelioration of one or more symptoms of the cancer. Preferably, the response may be reflected by decreased or diminished indices of the metastatic conversion of the breast cancer such as the prevention of the formation of metastases or a reduction of number or size of metastases *(see, e.g.,* Eisenhauser et al., New response evaluation criteria in solid tumours: Revised RECIST guideline (version 1.1) Eur. J. Cancer 2009 45: 228-247).

The phrase "sensitive to" in the context of the present invention indicates that a subject/patient suffering, suspected to suffer or prone to suffer from breast cancer, in particular locally advanced, recurrent or metastatic HER-2 negative breast cancer , shows in some way a positive reaction to treatment with bevacizumab in combination with a chemotherapy regimen. The reaction of the patient may be less pronounced when compared to a patient "responsive to" as described hereinabove. For example, the patient may experience less suffering associated with the disease, though no reduction in tumor growth or metastatic indicator may be measured, and/or the reaction of the patient to the bevacizumab in combination with the chemotherapy regimen may be only of a transient nature, *i.e.,* the growth of (a) tumor and/or (a) metastasis(es) may only be temporarily reduced or halted.

The phrase "a patient suffering from" in accordance with the invention refers to a patient showing clinical signs of breast cancer, in particular locally advanced, recurrent or metastatic HER-2 negative breast cancer . The phrases "suspected to suffer from", "being susceptible to", "prone to suffer from" or "being prone to", in the context of breast cancer, in particular locally advanced, recurrent or metastatic HER-2 negative breast cancer, refers to an indication disease in a patient based on, *e.g.,* a possible genetic predisposition, a pre- or eventual exposure to hazardous and/or carcinogenic compounds, or exposure to carcinogenic physical hazards, such as radiation.

The phrase "treatment effect of a chemotherapy regimen" in the context of the present invention encompasses the terms "overall survival" and "progression-free survival".

The phrase "overall survival" in the context of the present invention refers to the length of time during and after treatment the patient survives. As the skilled person will appreciate, a patient's overall survival is improved or enhanced, if the patient belongs to a subgroup of patients that has a statistically significant longer mean survival time as compared to another subgroup of patients.

The phrase "progression-free survival" in the context of the present invention refers to the length of time during and after treatment during which, according to the assessment of the treating physician or investigator, the patient's disease does not become worse, *i.e.,* does not progress. As the skilled person will appreciate, a patient's progression-free survival is improved or enhanced if the patient experiences a longer length of time during which the disease does not progress as compared to the average or mean progression free survival time of a control group of similarly situated patients.

The terms "administration" or "administering" as used herein mean the administration of an angiogenesis inhibitor, e.g., bevacizumab (Avastin®), and/or a pharmaceutical composition/treatment regimen comprising an angiogenesis inhibitor, e.g., bevacizumab (Avastin®), to a patient in need of such treatment or medical intervention by any suitable means known in the art for administration of a therapeutic antibody. Nonlimiting routes of administration include by oral, intravenous, intraperitoneal, subcutaneous, intramuscular, topical, intradermal, intranasal or intrabronchial administration (for example as effected by inhalation). Particularly preferred in context of this invention is parenteral administration, e.g., intravenous administration.

The term "antibody" is herein used in the broadest sense and includes, but is not limited to, monoclonal and polyclonal antibodies, multispecific antibodies (e.g., bispecific antibodies), chimeric antibodies, CDR grafted antibodies, humanized antibodies, camelized antibodies, single chain antibodies and antibody fragments and fragment constructs, e.g., $F(ab')_2$ fragments, Fab-fragments, Fv-fragments, single chain Fv-fragments (scFvs), bispecific scFvs, diabodies, single domain antibodies (dAbs) and minibodies, which exhibit the desired biological activity, in particular, specific binding to one or more of VEGFA, VEGFR2 and PLGF, or to homologues, variants, fragments and/or isoforms thereof.

The term "aptamer" is herein used in the broadest sense and includes, but is not limited to, oligonucleotides, including RNA, DNA and RNA/DNA molecules, or peptide molecules, which exhibit the desired biological activity, in particular, specific binding to one or more of VEGFA, VEGFR2 and PLGF, or to homologues, variants, fragments and/or isoforms thereof.

As used herein "chemotherapy regimen" or "chemotherapeutic agent" include any active agent that can provide an anticancer therapeutic effect and may be a chemical agent or a biological agent, in particular, that are capable of interfering with cancer or tumor cells. Preferred active agents are those that act as anti-neoplastic (chemotoxic or chemostatic) agents which inhibit or prevent the development, maturation or proliferation of malignant cells. Nonlimiting examples of a chemotherapy regimen or chemotherapeutic agents include alkylating agents such as nitrogen mustards (*e.g.*, mechlorethamine, cyclophosphamide, ifosfamide, melphalan and chlorambucil), nitrosoureas (*e.g.*, carmustine (BCNU), lomustine (CCNU), and semustine (methyl-CCNU)), ethylenimines/ methylmelamines (*e.g.*, thriethylenemelamine (TEM), triethylene, thiophosphoramide (thiotepa), hexamethylmelamine (HMM, altretamine)), alkyl sulfonates (*e.g.*, busulfan), and triazines *(e.g.,* dacarbazine (DTIC)); antimetabolites such as folic acid analogs (*e.g.,* methotrexate, trimetrexate), pyrimidine analogs (*e.g.*, 5-fluorouracil, capecitabine, fluorodeoxyuridine, gemcitabine, cytosine arabinoside (AraC, cytarabine), 5-azacytidine, 2,2'-difluorodeoxycytidine), and purine analogs (e.g., 6-mercaptopurine, 6-thioguanine, azathioprine, 2'-deoxycoformycin (pentostatin), erythrohydroxynonyladenine (EHNA), fludarabine phosphate, and 2-chlorodeoxyadenosine (cladribine, 2-CdA)); antimitotic drugs developed from natural products (*e.g.,* paclitaxel, vinca alkaloids (*e.g.,* vinblastine (VLB), vincristine, and vinorelbine), docetaxel, estramustine, and estramustine phosphate), epipodophylotoxins (.*e.g.,* etoposide, teniposide), antibiotics (.*e.g* actimomycin D, daunomycin (rubidomycin), daunorubicon, doxorubicin, epirubicin, mitoxantrone, idarubicin, bleomycins, plicamycin (mithramycin), mitomycinC, actinomycin), enzymes (*e.g.,* L-asparaginase), and biological response modifiers (*e.g.,* interferon-alpha, IL-2, G-CSF, GM-CSF); miscellaneous agents including platinum coordination complexes (*e.g.*, cisplatin, carboplatin, oxaliplatin), anthracenediones (*e.g.*, mitoxantrone), substituted urea (*i.e.,* hydroxyurea), methylhydrazine derivatives (*e.g.,* N-methylhydrazine (MIH), procarbazine), adrenocortical suppressants (*e.g.*, mitotane (o,p'-DDD), aminoglutethimide); hormones and antagonists including adrenocorticosteroid antagonists (.e.g prednisone and equivalents, dexamethasone, aminoglutethimide), progestins (e.g., hydroxyprogesterone caproate, medroxyprogesterone acetate, megestrol acetate), estrogens (*e.g.*, diethylstilbestrol, ethinyl estradiol and equivalents thereof); antiestrogens (*e.g.,* tamoxifen), androgens (*e.g.,* testosterone propionate, fluoxymesterone and equivalents thereof), antiandrogens (e.g., flutamide, gonadotropin-releasing hormone analogs, leuprolide), non-steroidal antiandrogens *(e.g.,* flutamide), epidermal growth factor inhibitors (*e.g.,* erlotinib, lapatinib, gefitinib) antibodies (e.g., trastuzumab), irinotecan and other agents such as leucovorin. For the treatment of locally advanced, recurrent or metastatic HER-2 negative breast cancer, chemotherapeutic agents for administration with bevacizumab include taxanes, such as pactitaxel and docetaxel (see also the illustrative example herein provided).

In the context of the present invention, "homology" with reference to an amino acid sequence is understood to refer to a sequence identity of at least 80%, particularly an identity of at least 85%, at least 90% or at least 95% over the full length of the sequence as defined by the SEQ ID NOs provided herein. In the context of this invention, a skilled person would understand that homology covers further allelic variation(s) of the marker/indicator proteins in different populations and ethnic groups.

As used herein, the term "polypeptide" relates to a peptide, a protein, an oligopeptide or a polypeptide which encompasses amino acid chains of a given length, wherein the amino acid residues are linked by covalent peptide bonds. However, peptidomimetics of such proteins/polypeptides are also encompassed by the invention wherein amino acid(s) and/or peptide bond(s) have been replaced by functional analogs, *e.g.*, an amino acid residue other than one of the 20 gene-encoded amino acids, *e.g.,* selenocysteine. Peptides, oligopeptides and proteins may be termed polypeptides. The terms polypeptide and protein are used interchangeably herein. The term polypeptide also refers to, and does not exclude, modifications of the polypeptide, e.g., glycosylation, acetylation, phosphorylation and the like. Such modifications are well described in basic texts and in more detailed monographs, as well as in a voluminous research literature. The term polypeptide also refers to and encompasses the term "antibody" as used herein.

The terms "treating" and "treatment" as used herein refer to remediation of, improvement of, lessening of the severity of, or reduction in the time course of the disease or any parameter or symptom thereof. Preferably said patient is a human patient and the disease to be treated is breast cancer, in particular locally advanced, recurrent or metastatic HER-2 negative breast cancer.

The terms "assessing" or "assessment" of such a patient relates to methods of determining the expression levels of one or more of the marker/indicator proteins described herein, including VEGFA, VEGFR2 and PLGF, and/or for selecting such patients based on the expression levels of such marker/indicator proteins relative to control levels established in patients diagnosed with breast cancer, in particular locally advanced, recurrent or metastatic HER-2 negative breast cancer.

The term "expression level" as used herein refers may also refer to the concentration or amount of marker/indicator proteins of the present invention in a sample.

In addition to the methods described above, the invention also encompasses further immunoassay methods for assessing or determining the expression level of one or more of VEGFA, VEGFR2 and PLGF, such as by Western blotting and ELISA-based detection. As is understood in the art, the expression level of the marker/indicator proteins of the invention may also be assessed at the mRNA level by any suitable method known in the art, such as Northern blotting, real time PCR, and RT PCR. Immunoassay- and mRNA-based detection methods and systems are well known in the art and can be deduced from standard textbooks, such as Lottspeich (Bioanalytik, Spektrum Akademisher Verlag, 1998) or Sambrook and Russell (Molecular Cloning: A Laboratory Manual, CSH Press, Cold Spring Harbor, NY, U.S.A., 2001). The described methods are of particular use for determining the expression levels of VEGFA, VEGFR2 and PLGF in a patient or group of patients relative to control levels established in a population diagnosed with breast cancer, in particular locally advanced, recurrent or metastatic HER-2 negative breast cancer.

The expression level of one or more of VEGFA, VEGFR2 and PLGF, can also be determined on the protein level by taking advantage of immunoagglutination, immunoprecipitation (*e.g.*, immunodiffusion, immunelectrophoresis, immune fixation), western blotting techniques (*e.g.,* (*in situ*) immuno cytochemistry, affinitychromatography, enzyme immunoassays), and the like. Amounts of purified polypeptide in solution may also be determined by physical methods, e.g. photometry. Methods of quantifying a particular polypeptide in a mixture usually rely on specific binding, *e.g.,* of antibodies. Specific detection and quantitation methods exploiting the specificity of antibodies comprise for example immunoassay methods. For example, concentration/amount of marker/indicator proteins of the present invention in a patient sample may be determined by enzyme linked-immunosorbent assay (ELISA). Alternatively, Western Blot analysis or immunostaining can be performed. Western blotting combines separation of a mixture of proteins by electrophoresis and specific detection with antibodies. Electrophoresis may be multidimensional such as 2D electrophoresis. Usually, polypeptides are separated in 2D electrophoresis by their apparent molecular weight along one dimension and by their isoelectric point along the other direction.

As mentioned above, the expression level of the marker/indicator proteins according to the present invention may also be reflected in an increased expression of the corresponding gene(s) encoding the VEGFA, VEGFR2 and/or PLGF. Therefore, a quantitative assessment of the gene product prior to translation (e.g. spliced, unspliced or partially spliced mRNA) can be performed in order to evaluate the expression of the corresponding gene(s). The person skilled in the art is aware of standard methods to be used in this context or may deduce these methods from standard textbooks (e.g. Sambrook, 2001, loc. cit.). For example, quantitative data on the respective concentration/amounts of mRNA encoding one or more of VEGFA, VEGFR2 and/or PLGF can be obtained by Northern Blot, Real Time PCR and the like.

In a further aspect of the invention, the kit of the invention may advantageously be used for carrying out a method of the invention and could be, inter alia, employed in a variety of applications, e.g., in the diagnostic field or as a research tool. The parts of the kit of the invention can be packaged individually in vials or in combination in containers or multicontainer units. Manufacture of the kit follows preferably standard procedures which are known to the person skilled in the art. The kit or diagnostic compositions may be used for detection of the expression level of one or more of VEGFA, VEGFR2 and PLGF in accordance with the herein-described methods of the invention, employing, for example, immunohistochemical techniques described herein.

Although exemplified by the use of bevacizumab, the invention encompasses the use of other angiogenesis inhibitors as known in the art for use in combination with standard chemotherapy regimens. The terms "angiogenesis inhibitor" as used herein refers to all agents that alter angiogenesis (e.g. the process of forming blood vessels) and includes agents that block the formation of and/or halt or slow the growth of blood vessels. Nonlimiting examples of angiogenesis inhibitors include, in addition to bevacizumab, pegaptanib, sunitinib, sorafenib and vatalanib. Preferably, the angiogenesis inhibitor for use in accordance with the methods of the present invention is bevacizumab. As used herein, the term "bevacizumab" encompass all corresponding anti-VEGF antibodies or anti-VEGF antibody fragments, that fulfil the requirements necessary for obtaining a marketing authorization as an identical or biosimilar product in a country or territory selected from the group of countries consisting of the USA, Europe and Japan.

For use in the detection methods described herein, the skilled person has the ability to label the polypeptides, for example antibodies, or oligonucleotides encompassed by the present invention. As routinely practiced in the art, hybridization probes for use in detecting mRNA levels and/or antibodies or antibody fragments for use in immunoassay methods can be labelled and visualized according to standard methods known in the art, nonlimiting examples of commonly used systems include the use of radiolabels, enzyme labels, fluorescent tags, biotin-avidin complexes, chemiluminescence, and the like.

The person skilled in the art, for example the attending physician, is readily in a position to administer the bevacizumab in combination with a chemotherapy regimen to the patient/patient group as selected and defined herein. In certain contexts, the attending physician may modify, change or amend the administration schemes for the bevacizumab and the chemotherapy regimen in accordance with his/her professional experience. Therefore, in certain aspects of the present invention, a method is provided for the treatment or improving the overall survival and/or progression-free survival of a patient suffering from or suspected to suffer from breast cancer, in particular locally advanced, recurrent or metastatic HER-2 negative breast cancer , with bevacizumab in combination with a chemotherapy regimen, whereby said patient/patient group is characterized in the assessment of a biological sample from the patient, in particular a blood plasma sample, said sample exhibiting an increased expression level of one or more of VEGFA, VEGFR2 and PLGF relative to control levels established in patients diagnosed with breast cancer, in particular locally advanced, recurrent or metastatic HER-2 negative breast cancer . The present invention also provides for the use of bevacizumab in the preparation of pharmaceutical composition for the treatment of a patient suffering from or suspected to suffer from breast cancer, in particular locally advanced, recurrent or metastatic HER-2 negative breast cancer, wherein the patients are selected or characterized by the herein disclosed protein marker/indicator status (*i.e.,* one or more of an increased expression level of VEGFA, VEGFR2 and PLGF relative to control levels established in patients diagnosed with breast cancer, in particular locally advanced, recurrent or metastatic HER-2 negative breast cancer.

The skilled person will recognize that the discovery that the expression level of one or more of VEGFA, VEGFR2 and PLGF in locally advanced, recurrent or metastatic HER-2 negative breast cancer patients is predictive of the sensitivity to or responsiveness of the patient to the addition of bevacizumab to a chemotherapy regimen may have implications for other cancers.

The figures show:

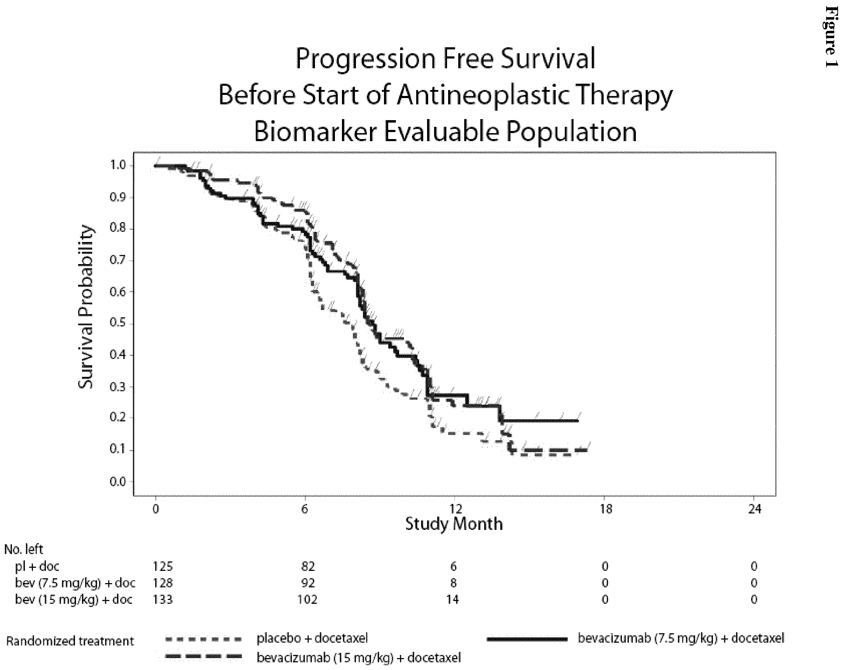

**Figure 1:** Kaplan Meier Curve for Progression Free Survival for the overall biomarker population for bevacizumab (low or high dose) plus docetaxel therapy versus placebo plus docetaxel therapy for patients being treated for locally advanced, recurrent or metastatic HER-2 negative breast cancer. Short-dash line represents placebo plus docetaxel. Solid line represents low dose bevacizumab (7.5 mg/kg every 3 weeks) plus docetaxel. Long-dash line represents high dose bevacizumab (15 mg/kg every 3 weeks) plus docetaxel.

**Figure 2:** Forest Plot of hazard ratio of progression-free survival before start of subsequent antineoplastic therapy by Biomarker (Placebo and Low Dose Bevacizumab), a dichotomized analysis, for bevacizumab (low dose) plus docetaxel therapy versus placebo plus docetaxel therapy for patients being treated for locally advanced, recurrent or metastatic HER-2 negative breast cancer.

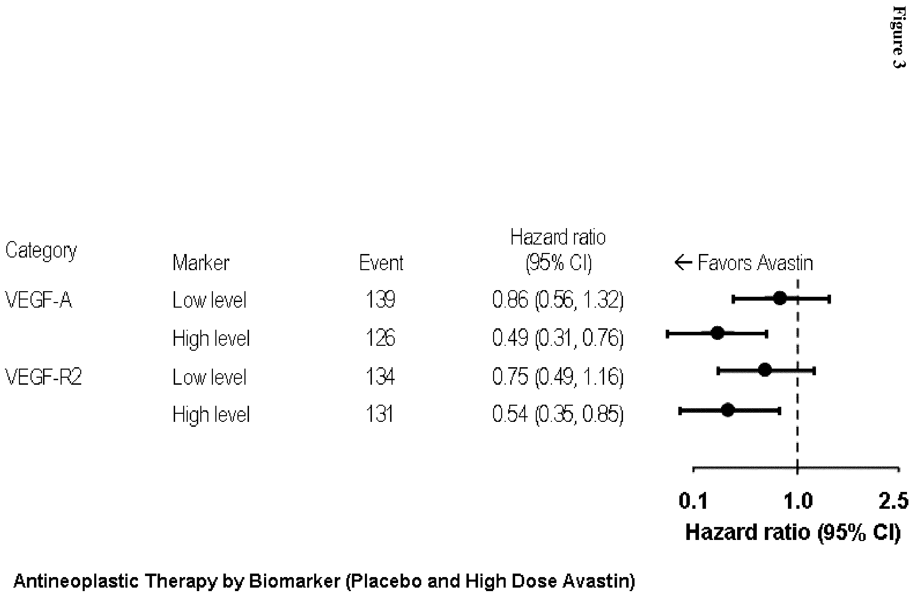

**Figure 3:** Forest Plot of hazard ratio of progression-free survival before start of subsequent antineoplastic therapy by Biomarker (Placebo and High Dose Bevacizumab), a dichotomized analysis, for bevacizumab (high dose) plus docetaxel therapy versus placebo plus docetaxel therapy for patients being treated for locally advanced, recurrent or metastatic HER-2 negative breast cancer.

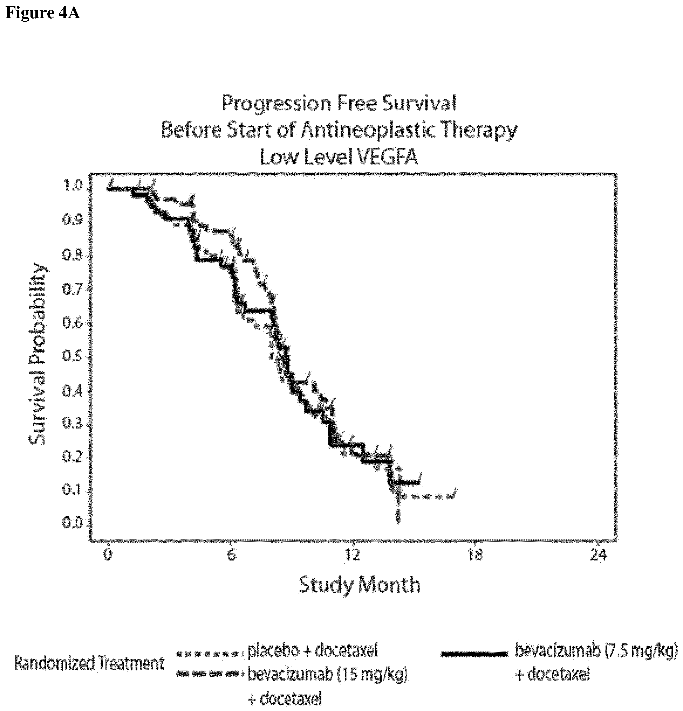

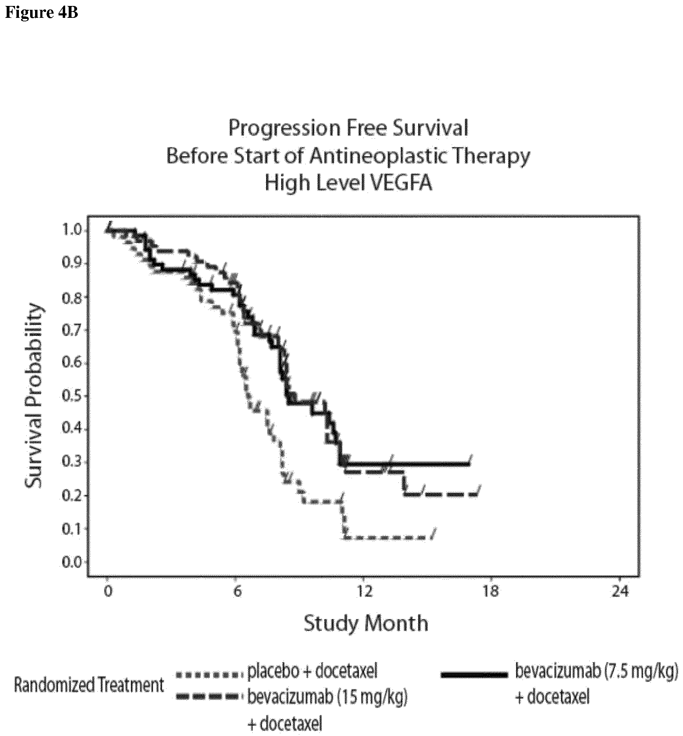

**Figure 4:** Kaplan Meier Curve of progression-free survival before start of subsequent antineoplastic therapy for low expression level (<125 pg/ml) VEGFA, (Figure 4A), and high expression level ($\geq$125 pg/ml) VEGFA, (Figure 4B), for bevacizumab (low or high dose) plus docetaxel therapy versus placebo plus docetaxel therapy for patients being treated for locally advanced, recurrent or metastatic HER-2 negative breast cancer. Short-dash line represents placebo plus docetaxel. Solid line represents low dose bevacizumab (7.5 mg/kg every 3 weeks) plus docetaxel. Long-dash line represents high dose bevacizumab (15 mg/kg every 3 weeks) plus docetaxel.

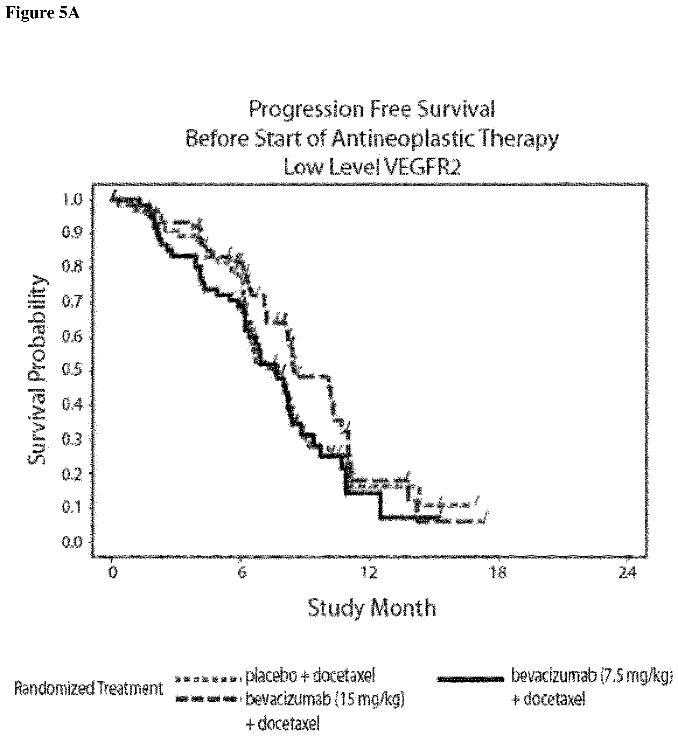

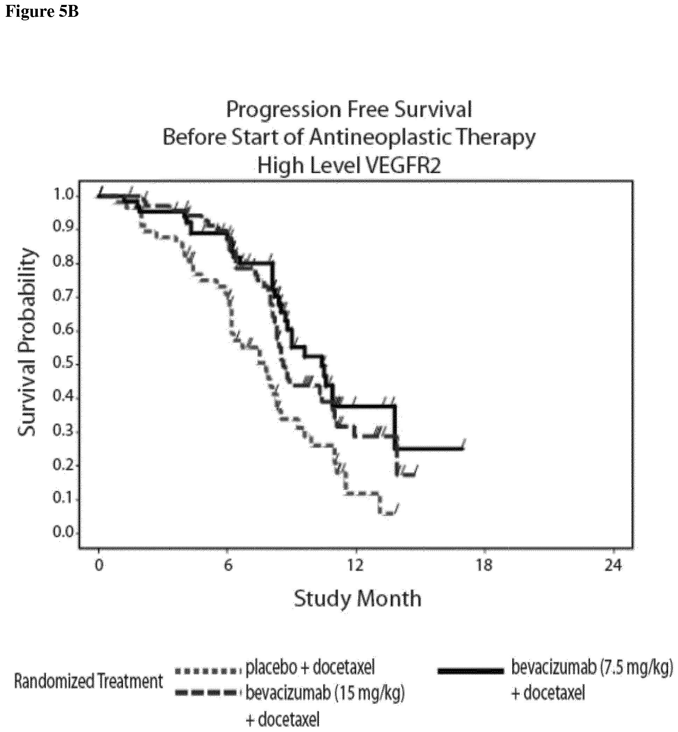

**Figure 5:** Kaplan Meier Curve of progression free survival before start of subsequent antineoplastic therapy for low expression level (<11 ng/ml) VEGFR2, (Figure 5A), and high expression level ($\geq$11 ng/ml) VEGFR2, (Figure 5B), for bevacizumab (low or high dose) plus docetaxel therapy versus placebo plus docetaxel therapy for patients being treated for locally advanced, recurrent or metastatic HER-2 negative breast cancer. Short-dash line represents placebo plus docetaxel. Solid line represents low dose bevacizumab (7.5 mg/kg every 3 weeks) plus docetaxel. Long-dash line represents high dose bevacizumab (15 mg/kg every 3 weeks) plus docetaxel.

**Figure 6:** Kaplan Meier Curve of progression free survival before start of subsequent antineoplastic therapy for combined low expression level (Formula 1 < -0.132) and combined high expression level (Formula 1 $\geq$ -0.132) of VEGFA and VEGFR2 for bevacizumab (low or high dose) plus docetaxel therapy versus placebo plus docetaxel therapy for patients being treated for locally advanced, recurrent or metastatic HER-2 negative breast cancer. Solid line represents placebo plus docetaxel. Long-dash represents low dose bevacizumab (7.5 mg/kg every 3 weeks) plus docetaxel. Short-dash line represents high dose bevacizumab (15 mg/kg every 3 weeks) plus docetaxel.

Figure 7: Kaplan Meier Curve of progression free survival before start of subsequent antineoplastic therapy for combined low expression level (Formula 2 < -0.006) and combined high expression level (Formula 2 $\geq$ -0.006) of VEGFA and PLGF for bevacizumab (low or high dose) plus docetaxel therapy versus placebo plus docetaxel therapy for patients being treated for locally advanced, recurrent or metastatic HER-2 negative breast cancer. Solid line represents placebo plus docetaxel. Long-dash line represents low dose bevacizumab (7.5 mg/kg every 3 weeks) plus docetaxel. Short-dash line represents high dose bevacizumab (15 mg/kg every 3 weeks) plus docetaxel.

**Figure 8:** SEQ ID NO: 1, Exemplary amino acid sequence of VEGFA.

**Figure 9:** SEQ ID NO:2, Exemplary amino acid sequence of VEGFR2.

**Figure 10:** SEQ ID NO:3, Exemplary amino acid sequence of PLGF.

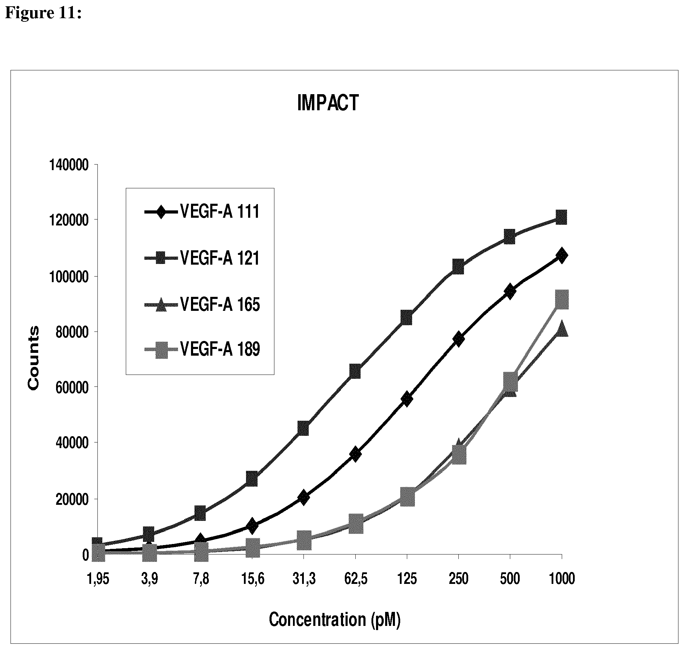

**Figure 11:** Measurements of increasing concentrations of $VEGF_{111}$, $VEGF_{121}$, $VEGF_{165}$ and $VEGF_{189}$ as measured on an IMPACT chip.

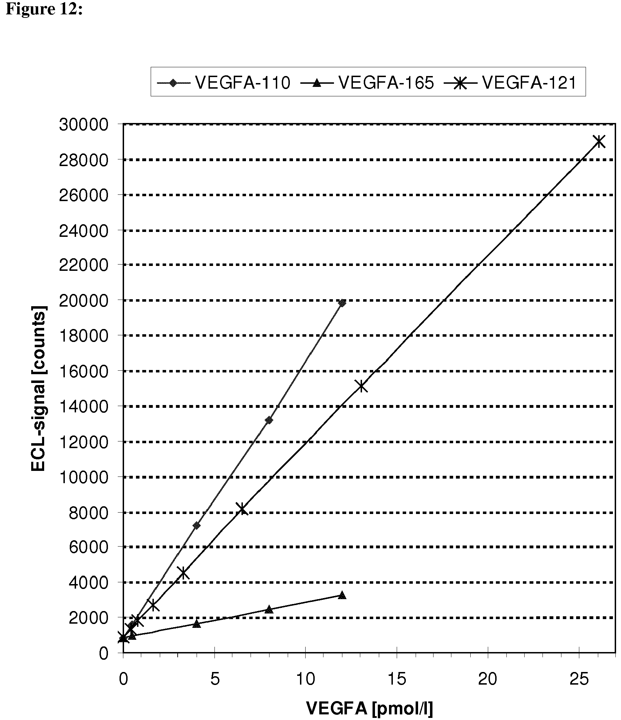

**Figure 12:** Measurements of increasing concentrations of $VEGF_{110}$, $VEGF_{121}$, and $VEGF_{165}$ as measured using the Elecsys® Assay on the automated Elecsys® analyzer.

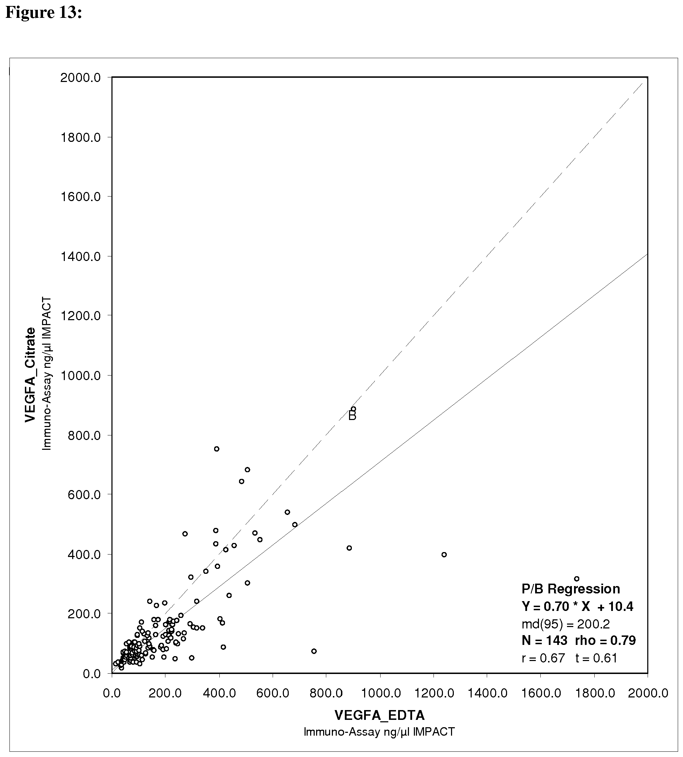

**Figure 13:** Data from EDTA- and Citrate samples from the same patients measured twice with the IMPACT assay. The VEGFA concentration is about 40% higher for EDTA-plasma than for Citrate with a Spearman correlation for the EDTA-Citrate method comparison of about 0.8.

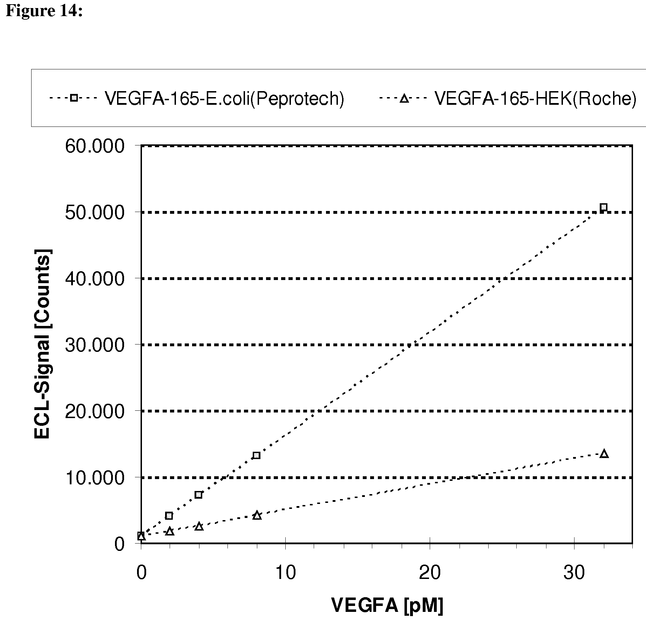

**Figure 14:** Shown are the counts (ECL-signal) measured when increasing concentrations of $VEGF_{165}$, produced recombinantly in E. coli or in HEK-cells, respectively, were measured on the automated Elecsys® analyzer.

The present invention is further illustrated by the following non-limiting illustrative example.

**EXAMPLE 1**

In the AVADO trial (BO17708) patients with untreated locally advanced, recurrent or metastatic HER-2 negative breast cancer were randomized to docetaxel 100 mg/m$^2$ plus bevacizumab 7.5 mg/kg every 3 weeks (n=248), bevacizumab 15 mg/kg (n=247) every three weeks or placebo (n=241) *see* Figure 1, *also see* Miles, J. Clin. Oncol., 24 May 2010 (e-published)).

Blood plasma baseline samples were available from 396 patients in this trial.

An investigation of the status of biomarkers related to angiogenesis and tumorigenesis revealed that the expression levels of three biomarkers relative to control levels determined in the entire biomarker patient population correlated with an improved treatment parameter. In particular, patients exhibiting a higher expression level of VEGFA relative to control levels determined in the entire biomarker patient population, demonstrated a prolonged progression free survival in response to the addition of bevacizumab to docetaxel therapy. Patients exhibiting a higher expression level of VEGFR2 relative to control levels determined in the entire biomarker patient population, demonstrated a prolonged progression free survival in response to the addition of bevacizumab to docetaxel therapy. Also patients exhibiting higher combined expression level of VEGFA and VEGFR2 relative to control levels determined in the entire biomarker patient population, demonstrated a prolonged progression free survival in response to the addition of bevacizumab to docetaxel therapy. In addition, patients exhibiting higher combined expression level of VEGFA and PLGF relative to control levels determined in the entire patient population, demonstrated a prolonged prolonged progression free survival in response to the addition of bevacizumab to docetaxel therapy.

**Patients and Immunochemical Methods**

A total of 736 patients participated in the BO17708 study, and blood plasma samples from 396 of the participants were available for biomarker analysis. The baseline characteristics of the 396 patients in the biomarker analysis and the remaining patients for which no biomarker analysis was possible are provided in Table 1A and Table 1B.

*Table 1A. Baseline characteristics*

|  | biomarker evaluable N=396 | biomarker unevaluable N=334 |
|---|---|---|
| Sex |  |  |
| Female | 396 (100%) | 334 (100%) |
| n | 396 | 334 |
|  |  |  |
| Randomized treatment |  |  |
| placebo + docetaxel | 129 (33%) | 109 (33%) |
| bevacizumab (7.5 mg/kg) + docetaxel | 129 (33%) | 118 (35%) |
| bevacizumab (15 mg/kg) + docetaxel | 138 (35%) | 107 (32%) |

(continued)

| | biomarker evaluable N=396 | biomarker unevaluable N=334 |
|---|---|---|
| n | 396 | 334 |
| | | |
| Age (years) | | |
| Mean | 54.4 | 52.8 |
| SD | 10.72 | 10.46 |
| SEM | 0.54 | 0.57 |
| Median | 55.0 | 53.0 |
| Min-Max | 29 - 83 | 26 - 77 |
| n | 396 | 334 |
| | | |
| Age Category (years) | | |
| <65 | 316 (80%) | 288 (86%) |
| > = 65 | 80 (20%) | 46 (14%) |
| n | 396 | 334 |
| | | |
| Race | | |
| White | 375 (95%) | 234 (70%) |
| Black | 4 (1%) | 3 (<1%) |
| Other | 17 (4%) | 97 (29%) |
| n | 396 | 334 |
| | | |
| Weight (kg) | | |
| Mean | 68.79 | 67.23 |
| SD | 14.097 | 14.185 |
| SEM | 0.708 | 0.780 |
| Median | 67.00 | 66.70 |
| Min-Max | 42.8 - 135.6 | 37.5 - 121.2 |
| n | 396 | 331 |
| | | |
| Height (cm) | | |
| Mean | 161.79 | 160.41 |
| SD | 7.158 | 7.351 |
| SEM | 0.360 | 0.402 |
| Median | 162.00 | 160.0 |
| Min-Max | 137.0 - 189.0 | 140.0 - 184.0 |
| n | 396 | 334 |
| | | |
| Smoking Status | | |

(continued)

|  | biomarker evaluable N=396 | biomarker unevaluable N=334 |
|---|---|---|
| Never smoked | 252 (64%) | 232 (70%) |
| Past smoker | 99 (25%) | 61 (18%) |
| Current smoker | 44 (11%) | 38 (11%) |
| n | 395 | 331 |
|  |  |  |
| Smoking - Pack Years |  |  |
| Mean | 24.06 | 33.63 |
| SD | 79.907 | 81.309 |
| SEM | 7.294 | 8.925 |
| Median | 10.00 | 15.00 |
| Min-Max | 0.3 - 860.0 | 0.5 - 720.0 |
| n | 120 | 83 |

*Table 1B. Baseline characteristics*

|  | biomarker evaluable N=396 | biomarker unevaluable N=334 |
|---|---|---|
| Body Surface Area (sqm) |  |  |
| Mean | 1.726 | 1.698 |
| SD | 0.1725 | 0.1796 |
| SEM | 0.0087 | 0.0099 |
| Median | 1.710 | 1.700 |
| Min-Max | 1.35 - 2.42 | 1.29 - 2.33 |
| n | 396 | 331 |
|  |  |  |
| Performance Status (ECOG) |  |  |
| 0 | 247 (63%) | 196 (60%) |
| 1 | 143 (37%) | 133 (40%) |
| 2 | 1 (<1%) | - |
| n | 391 | 329 |
|  |  |  |
| LVEF |  |  |
| < = median (64) | 181 (35%) | 172 (55%) |
| > median (64) | 177 (49%) | 138 (45%) |
| n | 358 | 310 |
|  |  |  |
| Disease Free Interval |  |  |
| < = 24 months | 138 (35%) | 118 (35%) |
| > 24 months | 255 (65%) | 216 (65%) |

(continued)

|  | biomarker evaluable N=396 | biomarker unevaluable N=334 |
|---|---|---|
| n | 393 | 334 |
|  |  |  |
| Hormone Receptor ER Status |  |  |
| Negative | 104 (26%) | 103 (31%) |
| Positive | 290 (73%) | 229 (69%) |
| Unknown | 2 (<1%) | 2 (<1%) |
| n | 396 | 334 |
|  |  |  |
| ER/PgR Combined Status |  |  |
| Negative | 81 (21%) | 82 (25%) |
| Positive | 314 (79%) | 250 (75%) |
| n | 395 | 332 |
|  |  |  |
| Number of Metastatic Sites |  |  |
| < 3 | 209 (53%) | 175 (53%) |
| >=3 | 183 (47%) | 156 (47%) |
| n | 392 | 331 |

**Blood Plasma Analysis**

Plasma samples were collected after randomization and before any study treatment was administered. All samples were obtained from patients that were thereafter treated with docetaxel 100mg/m2 plus either bevacitumab 7.5mg/kg every three weeks, bevacizumab 15mg/kg every three weeks or placebo until disease progression.

A total of 4.9 mLs of blood were drawn into a S-monovette® (EDTA) tube (or a citrated plasma tube for the 16 patients on anticoagulant therapy). They were mixed immediately thereafter by gentle invertion of the tube and were centrifuged within 30 minutes at approximately 1500g in centrifuge (room temperature for 10 minutes). Immediately hereafter, supernatant plasma was aliquoted in a clear polypropylene 5mL transfer tube. Thereafter, plasma was aliquoted into 2 plastic storage tubes (approximately 1.25 ml each). Samples were stored in an upright position at -70°C. In some cases, samples were stored at - 20°C for up to one month and then transferred to -70°C.

Samples were used for measurement of levels of VEGFA, VEGF receptor-1 (VEGFR1), VEGFR2, PLGF and E-SELECTIN using an Immunological MultiParameter Chip Technology (IMPACT) from Roche Diagnostics GmbH.

IMPACT Multiplex Assay Technology

Roche Professional Diagnostics (Roche Diagnostics GmbH) has developed a multimarker platform under the working name IMPACT (Immunological MultiParameter Chip Technology). This technology was used for the measurement of the protein markers mentioned above in the "blood plasma analysis" section. The technology is based on a small polystyrene chip manufactured by procedures as disclosed in EP 0939319 and EP 1610129. The chip surface was coated with a streptavidin layer, onto which the biotinylated antibodies were then spotted for every assay. For each marker, spots of antibodies were loaded in a vertical line onto the chip. During the assay, the array was probed with specimen samples containing the specific analytes.

For the described analyses, chips from Lot01 were used

The plasma volume required per specimen for measuring all markers on one chip was 8 $\mu$L, which was applied together with 32 $\mu$L of an incubation buffer (50 mM HEPES pH 7.2, 150 mM NaCl, 0.1% Thesit, 0.5% bovine serum albumin and 0.1% Oxypyrion as a preservative agent). After incubation for 12 minutes and washing of the chip using a washing buffer (5 mM Tris pH 7.9, 0.01% Thesit and 0.001% Oxypyrion) the digoxigenylated monoclonal antibody mix was added (40 µL of incubation buffer including a mix of the analyte-specific antibodies labeled with Digoxigenin) and was incubated for an additional 6 minutes to bind onto the captured analytes. The second antibody was finally detected with 40 µL of a reagent buffer (62.5 mM TAPS pH 8.7, 1.25 M NaCl, 0.5% bovine serum albumin, 0.063% Tween 20 and 0.1% Oxypyrion) including an anti-digoxigenin antibody conjugate coupled with fluorescent latex. Using this label, 10 individual binding events in a single spot could be detected, resulting in very high sensitivity down to the fmol/L concentration. Chips were transported into the detection unit, and a charge coupled device (CCD) camera generated an image that was transformed into signal intensities using dedicated software. Individual spots were automatically located at predefined positions and quantified by image analysis. For each marker, lines of 10-12 spots were loaded on the chips, and a minimum of 5 spots was required to determine the mean concentration of samples. The advantages of the technology are the ability of multiplexing up to 10 parameters in a sandwich or competitive format. The calibrators and patient samples were measured in duplicate. One run was designed to contain a total of 100 determinations, including 2 multi-controls as a run control. Since some of the selected analytes react with each other (i.e VEGFA and PLGF with VEGFR1 or VEGRF2 or VEGFA forms heterodimers with PLGF), the 5 analytes were divided on three different chips as follows:

Chip 1: VEGFA
Chip 2: VEGFR1, VEGFR2, E-Selectin
Chip 3: PLGF

The following antibodies were used for the different assays:

| Analyte | Capture antibody | Manufacturer | Detection antibody | Manufacturer |
|---|---|---|---|---|
| VEGFA | <VEGF-A>M-3C5 | Bender RELIATech | <VEGF>M-26503 | R&D Systems |
| VEGFR1 | <VEGF-R1>M-49560 | Roche Diagnostics | <VEGF-R1>M-49543 | Roche Diagnostics |
| VEGFR2 | <VEGF-R2>M-89115 | R&D Systems | <VEGF-R2>M-89109 | R&D Systems |
| E-Selectin | <E-Selectin>M-BBIG-E5 | R&D Systems | <E-Selectin>M-5D11 | R&D Systems |
| PLGF | <PLGF>M-2D6D5 | Roche Diagnostics | <PLGF>M-6A11D2 | Roche Diagnostics |

## Statistical Analysis

Sample median was used to dichotomize biomarker values as low (below median) or high (at or above median).
Hazard Ratio of treatment effect in sub-group of patients with high or low biomarker levels were estimated with proportional hazard cox regression analysis.
In addition, proportional hazard cox regressions was used to evaluate the association between biomarker level and treatment effect. The model included the following covariates : trial treatment, biomarker level, binary stratification factors (ER/PgR status, measurable disease at baseline, prior adjuvant taxane therapy), interaction term of treatment by biomarker level. Wald test for the interaction term was used to determine the association between biomarker level and treatment effect. P-value below 0.05 was considered significant.

## Results

Blood Plasma Markers

The baseline descriptive statistics of the biomarkers are presented in Table 2.

*Table 2: Descriptive Statistics of Biomarker Values (Baseline)*

| | *VEGFA* (pg/mL) | *VEGFR2* (ng/mL) | *PLGF* (pg/mL) |
|---|---|---|---|
| *min* | 20.0 | 0.1 | 5.8 |
| *qu 25%* | 64.5 | 9.1 | 17.04 |
| *median* | 125.0 | 11.0 | 21.31 |
| *qu 75%* | 240.5 | 13.4 | 27.02 |

(continued)

| | VEGFA (pg/mL) | VEGFR2 (ng/mL) | PLGF (pg/mL) |
|---|---|---|---|
| max | 3831.1 | 72.4 | 282.10 |
| mean | 216.5 | 11.6 | 24.58 |
| sd | 322.63 | 4.58 | 20.38 |

Table 3 presents the results of the analysis of the association of VEGFA or VEGFR2 with treatment effect on progression free survival.

**Table 3**

| | Low dose | | High dose | |
|---|---|---|---|---|
| | HR (95%CI) | Interaction p-value | HR (95%CI) | Interaction p-value |
| VEGFA low | 0.96 (0.62 - 1.48) | P=0.0136 | 0.86 (0.56 - 1.32) | P=0.0808 |
| VEGFA high | 0.52 (0.33 - 0.81) | | 0.49(0.31 - 0.76) | |
| VEGFR2 low | 1.10 (0.73 - 1.67) | P=0.0342 | 0.75 (0.49 - 1.16) | P=0.2545 |
| VEGFR2 high | 0.46 (0.28 - 0.74) | | 0.54 (0.35 - 0.85) | |

In this analysis, for VEGFA, Low VEGFA (<125 pg/ml) and High VEGFA ($\geq$125 pg/ml), and for VEGFR2, Low VEGFR2 (<11 ng/ml) and High VEGFR2 ($\geq$11 ng/ml) were used.

These results show that the Hazard Ratio for treatment effect is significantly better in the subset of patients with high VEGFA compared to patients with low VEGFA. These results also show that the Hazard Ratio for treatment effect is significantly better in the subset of patients with high VEGFR2 compared to patients with low VEGFR2. The same trend is observed when comparing low and high dose bevacizumab to placebo, the statistical evidence of difference between high and low biomarker sub-group is stronger in the patients treated with low dose bevacizumab. Therefore, VEGFA and VEGFR2 are each independent predictive biomarkers for bevacizumab treatment effect on Progression Free Survival.

Table 4 presents the analysis of biomarker combinations association with treatment effect on progression free survival for low dose (7.5 mg/kg every 3 weeks) bevacizumab and for high dose (15 mg/kg every 3 weeks) bevacizumab.

For this analysis

Formula 1: norm(VEGFA)+1.3*norm(VEGFR2)
Equivalent formula: 0.71*log2(VEGFA)+3.16*log2(VEGFR2)-15.6
and
Formula 2: 0.25*norm(VEGFA)+0.21*norm(PLGF)
Equivalent formula: 0.18*log2(VEGFA)+0.42*log2(PLGF)-3.1

Where we use log2 transformation and

$$x_i \rightarrow norm(x_i) = \frac{\log 2(x_i) - median(\log 2(x))}{mad(\log 2(x))}$$

Where mad is the median absolute deviation adjusted by a factor of 1.4826.

*Table 4: Association with treatment effect on Progression Free Survival (bi-marker analysis) for low dose (7.5 mg/kg every 3 weeks) bevacizumab and for high dose (15 mg/kg every 3 weeks) bevacizumab*

|  | Low Dose (7.5 mg/kg) versus Placebo | | High Dose (15 mg/kg) versus Placebo | |
|---|---|---|---|---|
|  | HR (95%CI) | Interaction p-value | HR (95%CI) | Interaction p-value |
| VEGFA & VEGFR2 low | 1.1 (0.72,1.69) | 0.0077 | 0.84 (0.54, 1.3) | 0.0580 |
| VEGFA & VEGFR2 high | 0.474 (0.3,0.75) | | 0.483 (0.31,0.76) | |
| VEGFA & PLGF low | 1.01 (0.65,1.58) | 0.037 | 0.845 (0.53,1.34) | 0.12 |
| VEGFA & PLGF high | 0.518 (0.33,0.81) | | 0.507 (0.33,0.78) | |

In this analysis, a high combined expression level of VEGFA and VEGFR2 is Formula 1 $\geq$ -0.132 and a low combined expression level of VEGFA and VEGFR2 is Formula 1 < -0.132, and a high combined expression level of VEGFA and PLGF is Formula 2 $\geq$ -0.006 and a low combined expression level of VEGFA and PLGF is Formula 2 < -0.006.

These results show that the Hazard Ratio for treatment effect is significantly better in the subset of patients with high VEGFA & VEGFR2 combination compared to patients with low VEGFA & VEGFR2 combination. These results also show that the Hazard Ratio for treatment effect is significantly better in the subset of patients with high VEGA & PLGF combination compared to patients with low VEGFA & PLGF combination. The same trend is observed when comparing low and high dose bevacizumab to placebo, the statistical evidence of difference between high and low biomarker sub-group is stronger in patients treated with low dose bevacizumab. Therefore, VEGFA & VEGFR2 combination and VEGFA & PLGF combination are each independent predictive biomarkers for bevacizumab treatment effect on Progression Free Survival.

The predictive value of VEGF-A in the bevacizumab 15 mg/kg arm was explored further by subdividing the cohort into quartiles according to VEGF-A levels. The 95% confidence intervals for all quartiles overlapped. In the first quartile (< 64 pg/ml), a very limited treatment effect was observed (hazard ratio 0.86). In the highest quartile (>240 pg/ml), the hazard ratio for PFS was 0.39 (95% CI:0.19-0.77) and te difference in median PFS was more pronounced than in the other groups. Overall, the point estimates of the quartiles show a consistent improvement in the hazard ratio with increasing VEGF-A levels. These results are shown in Table 5 below.

*Table 5: PFS According to VEGF-A Quartile*

| Median PFS Months | | | | | |
|---|---|---|---|---|---|
| VEGF-A Quartile | No. of patients | No. of Events | Bevacizumab 15 mg/kg + docetaxel | Placebo + docetaxel | HR (95% CI) |
| 1st | 71 | 43 | 8.6 | 8.3 | 0.86 (0.47-1.59) |
| 2nd | 68 | 43 | 8.5 | 7.2 | 0.75 (0.42-1.44) |
| 3rd | 65 | 43 | 8.4 | 6.5 | 0.55 (0.30-1.01) |
| 4th | 61 | 36 | 10.3 | 7.5 | 0.39 (0.19-0.77) |

**Example 2: Detection of shorter isoforms of VEGF-A using the IMPACT Assay**

This example demonstrates that, based on the antibodies used for detection of VEGF-A on the IMPACT platform, the shorter isoforms of VEGF-A are preferentially measured as compared to the longer isoforms of VEGF-A.

The assay was performed as described above under the section relating to the IMPACT technology using the antibodies listed in the table before the "statistical analysis" section.

Four different VEGF-A forms, i.e. $VEGF_{111}$, $VEGF_{121}$, $VEGF_{165}$ and $VEGF_{189}$ were available and used in the

analysis. $VEGF_{111}$, $VEGF_{121}$ (both derived from expression in E. coli), and $VEGF_{165}$ (obtained recombinantly in an insect cell line) was purchased from R&D Systems, Minneapolis, USA and $VEGF_{189}$ was obtained from RELIATech, Wolfenbüttel, Germany. It has turned out later that VEGF189 appears to be rather unstable and that the data obtained with that material cannot be relied upon. As shown in Figure 11 the shorter isoforms having 111 or 121 amino acids, respectively, which had been produced in E. coli and are not secondarily modified, e.g., not glycosylated, are detected better as compared to the longer isoforms with 165 amino acids. VEGF165 had been obtained in an insect cell line and is at least partially glycosylated. The biologically interesting plasmin cleavage product $VEGF_{110}$ was not available for testing at this point in time, but is has to be expected that detection of this isoform will be comparable to what is seen for the VEGF-molecule with 111 amino acids.

**Example 3: Detection of short VEGF isoforms using the Elecsys® Analyzer**

This example describes experiments demonstrating that an assay using the Elecsys® Analyzer and a corresponding assay can be used to detect short VEGF isoforms in human plasma.

The VEGF-A assay was transferred from IMPACT to the automated in-vitro diagnostics system Elecsys® (Roche Diagnostics GmbH, Mannheim). The same capture antibody as in the IMPACT Assay, <hVEGF-A>-m3C5 (RELIATech, Wolfenbüttel) was used, while the capture antibody <hVEGF-A>-m25603 (R&D Systems, Minneapolis) used on the IMPACT system was replaced by <hVEGF-A>-mA4.6.1 (Genentech, South San Francisco).

The immunoassays running on the automated Elecsys® system are immuno assays using electrochemiluminescense (ECLIA) as the signal generating technology. In the present sandwich assay the biotinylated capture antibody binds to streptavidin coated, magnetic microparticles and the ruthenylated detection antibody allows for signal generation. 75 µl of biotinylated <VEGF-A>-m3C5 at 1.5 µg/ml and 75 µl of ruthenylated <VEGF-A>M-A.4.6.1 at 2 µg/ml both in reaction buffer (50 mM Tris (pH 7.4), 2 m M EDTA, 0.1 % thesit, 0.2 % bovine IgG, 1.0 % bovine serum albumin) were incubated for 9 minutes with 20 µl of sample. 30 µl of a microparticle suspension was added after the first 9 minutes of incubation and the whole mixture then incubated for an additional 9 minutes. During these incubation steps an antibody analyte antibody sandwich is formed that is bound to the microparticles. Finally the microparticles were transferred to the detection chamber of the Elecsys system for signal generation and readout.

The cleavage product/isoform preference of the Elecsys® VEGF-A assay was assessed with purified recombinant proteins: VEGF 110 (produced by plasmin cleavage at Genentech, South San Francisco), VEGF 121 and VEGF 165 (both produced in an insect cell line and supplied by R&D Systems, Minneapolis). The preferential binding of short VEGF isoforms that had been seen with the IMPACT® Assay was confirmed in the Elecsys assay. As shown in Figure 12, in the Elecsys® assay the isoforms VEGF 121 and the plasmin cleavage product VEGF 110, respectively, both were detected with an approximately 5-fold higher sensitivity than VEGF 165.

**Example 4: Detection of short VEGF isoforms in plasma collected in Na citrate and EDTA**

Paired plasma samples were collected from patients with HER2+ locally recurrent or metastatic breast cancer in both an EDTA monovette (5mL)- and Citrate Monovette collection tube (5mL). Within 30 minutes of blood collection, blood tubes were placed into the centrifuge and spun 1500 g at room temperature for 10 minutes, until cells and plasma were separated. Immediately after centrifugation, the plasma was carefully transferred into a propylene transfer tube and then aliquotted equally into 2 storage tubes (half volume each approximately 1.25 mL) using a pipette. The levels of VEGF-A in the samples were measured using the IMPACT Assay described above. As shown in Figure 13, the VEGFA concentration is about 40% higher for plasma samples collected and stored in EDTA compared to plasma samples collected and stored in citrate with a Spearman correlation for the EDTA-Citrate MC of about 0.8 for baseline samples collected prior to treatment.

**Example 5: Comparative measurement of unmodified and modified VEGF165 on the Elecsys analyzer**

This example describes experiments demonstrating that that the Elecsys® Analyzer and a corresponding assay can be used to detect unmodified VEGF in human plasma.

The VEGF-A assay was transferred from IMPACT to the automated in-vitro diagnostics system Elecsys® (Roche Diagnostics GmbH, Mannheim). The same capture antibody as in the IMPACT assay, <hVEGF-A>-m3C5 (RELIATech GmbH, Wolfenbüttel) was used, while the detection antibody <hVEGF-A>-m25603 (R&D Systems, Minneapolis) used on the IMPACT system was replaced by <hVEGF-A>-mA4.6.1 (Genentech, South San Francisco).

The immunoassays running on the automated Elecsys system are immuno assays using electrochemiluminescense (ECLIA) as the signal generating technology. In the present sandwich assay the biotinylated capture antibody binds to streptavidin coated, magnetic microparticles and the ruthenylated detection antibody allows for signal generation. 75 µl of biotinylated <VEGF-A>-m3C5 at 1.5 µg/ml and 75 µl of ruthenylated <VEGF-A>M-A.4.6.1 at 2 µg/ml both in reaction buffer (50 mM Tris (pH 7.4), 2 m M EDTA, 0.1 % thesit, 0.2 % bovine IgG, 1.0 % bovine serum albumin) were incubated for 9 minutes with 20 $\mu$l of sample. 30 $\mu$l of a microparticle suspension was added after the first 9 minutes of incubation and the whole mixture then incubated for an additional 9 minutes. During these incubation steps an antibody-analyte-antibody sandwich is formed that is bound to the microparticles. Finally the microparticles were transferred to the detection chamber of the Elecsys system for signal generation and readout.

The preference of the Elecsys VEGF-A assay was assessed with purified recombinant proteins: VEGF165 (produced recombinantly in E. coli by Peprotech) and VEGF165 (produced recombinantly in HEK-cells at Roche Diagnostics, Germany). The preferential binding of unmodified VEGF165 that had been seen with the IMPACT assay was confirmed in the Elecsys assay. As shown in Figure 14, in the Elecsys assay the unmodified VEGF 165 was detected with an approximately 5-fold higher sensitivity than modified VEGF 165.

Furthermore, the present invention relates to the following items:

1. A method for improving the progression free survival of a patient suffering from breast cancer by adding bevacizumab to said chemotherapy regimen, said method comprising:

(a) determining the protein expression level of VEGFA and/or VEGFR2 in a patient sample; and
(b) administering bevacizumab in combination with a chemotherapy regimen to the patient having an increased expression level of VEGFA and/or VEGFR2 relative to control expression levels determined in patients diagnosed with breast cancer.

2. An in vitro method for the identification of a patient responsive to or sensitive to the addition of bevacizumab treatment to a chemotherapy regimen, said method comprising determining the protein expression level of VEGFA and/or VEGFR2 in a sample from a patient suspected to suffer from or being prone to suffer from breast cancer, whereby an increased expression level of VEGFA and/or VEGFR2 relative to control expression levels determined in patients suffering from breast cancer is indicative of a sensitivity of the patient to the addition of bevacizumab to said chemotherapy regimen.

3. Use of bevacizumab for improving the progression free survival of a chemotherapy regimen of a patient suffering from breast cancer comprising the following steps:

(a) determining the protein expression level of VEGFA and/or VEGFR2 in a patient sample; and
(b) administering bevacizumab in combination with a chemotherapy regimen to the patient having an increased expression level of VEGFA and/or VEGFR2 relative to control expression levels determined in patients diagnosed with breast cancer.

4. The method or use of any one of items 1 to 3, wherein the dose of bevacizumab administered is low dose bevacizumab.

5. The method or use of item 4, wherein the low dose bevacizumab is 7.5 mg/kg of body weight every 3 weeks.

6. The method or use of any one of items 1 to 3, wherein the dose of bevacizumab administered is high dose bevacizumab.

7. The method or use of item 6, wherein the high dose bevacizumab is 15 mg/kg of body weight every 3 weeks.

8. The method of use of item 6, wherein the high dose bevacizumab is 10 mg/kg of body weight every 2 weeks.

9. An in vitro method of predicting the response to or sensitivity to the addition of bevacizumab to a chemotherapy regimen of a patient suspected to suffer from, suffering from or prone to suffer from breast cancer comprising determining the protein expression level of VEGFA and/or VEGFR2 in a patient sample.

10. The method or use of any one of items 1 to 9, wherein the protein expression level determined is a combined expression level of VEGFA and VEGFR2.

11. The method or use of any one of items 1 to 5 and 9, wherein the protein expression level determined is a combined expression level of VEGFA and VEGFR2.

12. The method or use of any one of items 1 to 9, further comprising determining the protein expression level of PLGF wherein the protein expression level determined is a combined expression level of VEGFA and PLGF.

13. Use of specific probes for the preparation of a diagnostic composition for predicting the response to or sensitivity to the addition of bevacizumab to a chemotherapy regimen of a patient suffering from, suspect to suffer from or prone to suffer from breast cancer comprising determining the expression level of one or more of VEGFA and VEGFR2 in a patient sample, wherein said probes are capable of detecting one or more of VEGFA and VEGFR2.

14. The use of item 13 further comprising a probe for determining the expression level of PLGF.

15. The use of item 13 or 14 wherein the probes are binding molecules like antibodies.

16. The method or use of any one of items 1 to 15, wherein said expression level is detected by an immunoassay method.

17. The method or use of item 16, wherein said immunoassay method is ELISA.

18. The method or use of any one of items 1 to 17, wherein said patient sample is a blood sample.

19. The method or use of any one of items 1 to 18, wherein said patient sample is a blood plasma sample.

20. The method or use of any one of items 1 to 19, wherein said breast cancer is locally advanced, recurrent or metastatic HER-2 negative breast cancer.

21. The method or use of any one of item 1 to 20, wherein said chemotherapy regimen comprises docetaxel or paclitaxel.

22. The method of use of any one of item 1 to 21, wherein said chemotherapy regimen comprises docetaxel.

23. The method or use of any one of items 1 to 22, wherein said patient is being co-treated with one or more anti-cancer therapies.

24. The method or use of item 23, wherein said anti-cancer therapy is radiation.

25. The method or use of any one of items 1 to 24, wherein said sample is obtained before neoadjuvant or adjuvant therapy.

26. The method or use of any one of items 1 to 24, wherein said samples is obtained after neoadjuvant or adjuvant therapy.

27. A kit useful for carrying out the method of any one of items 1 to 26, comprising polypeptides capable of determining the expression level of one or more of VEGFA, VEGFR2 and/or PLGF.

28. Use of a polypeptide for determining the expression level of one or more of VEGFA, VEGFR2 and/or PLGF in any one of items 1 to 26.

29. The kit of item 27 or the use of item 28 comprising a polypeptide capable of determining the expression level of one or more of VEGFA, VEGFR2 and/or PLGF, wherein said polypeptide is suitable for use in an immunoassay method and/or is an antibody specific for VEGFA, VEGFR2 or PLGF.

**Claims**

1. An in vitro method for the identification of a patient responsive to or sensitive to the addition of bevacizumab treatment to a chemotherapy regimen, said method comprising determining the protein expression level of VEGFR2 in a blood plasma sample from a patient suspected to suffer from or being prone to suffer from breast cancer, whereby an increased expression level of VEGFR2 relative to control expression levels determined in patients suffering from breast cancer is indicative of a sensitivity of the patient to the addition of bevacizumab to said chemotherapy regimen.

2. The method of claim 1, wherein the dose of bevacizumab administered is low dose bevacizumab.

3. The method of claim 2, wherein the low dose bevacizumab is 7.5 mg/kg of body weight every 3 weeks.

4. The method of claim 1, wherein the dose of bevacizumab administered is high dose bevacizumab.

5. The method of claim 4, wherein the high dose bevacizumab is 15 mg/kg of body weight every 3 weeks or 10 mg/kg of body weight every 2 weeks.

6. An in vitro method of predicting the response to or sensitivity to the addition of bevacizumab to a chemotherapy regimen of a patient suspected to suffer from, suffering from or prone to suffer from breast cancer comprising determining the protein expression level of VEGFR2 in a patient blood plasma sample.

7. The method of claim 1 to 6, wherein said expression level is detected by an immunoassay method.

8. The method or use of claim 7, wherein said immunoassay method is ELISA.

9. The method of claim 1 to 8, wherein said breast cancer is locally advanced, recurrent or metastatic HER-2 negative breast cancer.

10. The method of claim 1 to 9, wherein said chemotherapy regimen comprises docetaxel or paclitaxel.

11. The method of claim 1 to 10, wherein said chemotherapy regimen comprises docetaxel.

12. The method of claim 1 to 11, wherein said patient is being co-treated with one or more anti-cancer therapies.

13. The method of claim 12, wherein said anti-cancer therapy is radiation.

14. The method of claim 1 to 13, wherein said sample is obtained before neoadjuvant or adjuvant therapy or after neoadjuvant or adjuvant therapy.

Figure 1

Progression Free Survival
Before Start of Antineoplastic Therapy
Biomarker Evaluable Population

| No. left | | | | | |
|---|---|---|---|---|---|
| pl + doc | 125 | 82 | 6 | 0 | 0 |
| bev (7.5 mg/kg) + doc | 128 | 92 | 8 | 0 | 0 |
| bev (15 mg/kg) + doc | 133 | 102 | 14 | 0 | 0 |

Randomized treatment      = = = = = = = =  placebo + docetaxel      ——————  bevacizumab (7.5 mg/kg) + docetaxel

= = = = = =  bevacizumab (15 mg/kg) + docetaxel

**Figure 2**

| Category | Marker | Event | Hazard ratio (95% CI) |
|---|---|---|---|
| VEGF-A | Low level | 127 | 0.96 (0.62, 1.48) |
| | High level | 128 | 0.52 (0.33, 0.81) |
| VEGF-R2 | Low level | 133 | 1.10 (0.73, 1.67) |
| | High level | 122 | 0.46 (0.28, 0.74) |

Antineoplastic Therapy by Biomarker (Placebo and Low Dose Avastin)

Figure 3

| Category | Marker | Event | Hazard ratio (95% CI) | ← Favors Avastin |
|----------|--------|-------|----------------------|------------------|
| VEGF-A | Low level | 139 | 0.86 (0.56, 1.32) | |
| | High level | 126 | 0.49 (0.31, 0.76) | |
| VEGF-R2 | Low level | 134 | 0.75 (0.49, 1.16) | |
| | High level | 131 | 0.54 (0.35, 0.85) | |

0.1     1.0     2.5

**Hazard ratio (95% CI)**

**Antineoplastic Therapy by Biomarker (Placebo and High Dose Avastin)**

**Figure 4A**

Progression Free Survival
Before Start of Antineoplastic Therapy
Low Level VEGFA

Randomized Treatment · · · · · · · · placebo + docetaxel ——— bevacizumab (7.5 mg/kg) + docetaxel
— — — bevacizumab (15 mg/kg) + docetaxel

**Figure 4B**

Progression Free Survival
Before Start of Antineoplastic Therapy
High Level VEGFA

Randomized Treatment
placebo + docetaxel
bevacizumab (15 mg/kg) + docetaxel
bevacizumab (7.5 mg/kg) + docetaxel

**Figure 5A**

Progression Free Survival
Before Start of Antineoplastic Therapy
Low Level VEGFR2

Figure 5B

Progression Free Survival
Before Start of Antineoplastic Therapy
High Level VEGFR2

Randomized Treatment
· · · · · · · · placebo + docetaxel
▬ ▬ ▬ ▬ bevacizumab (15 mg/kg) + docetaxel
▬▬▬ bevacizumab (7.5 mg/kg) + docetaxel

**Figure 6**

Bevacizumab effect in patients with low VEGFA & VEGFR2
Progression Free Survival

Bevacizumab effect in patients with high VEGFA & VEGFR2
Progression Free Survival

**Figure 7**

Bevacizumab effect in patients with low VEGFA & PLGF
Progression Free Survival

placebo    n=61/36, med=254
low dose   n=68/42, med=252
high dose        n=68/40, med=258

Bevacizumab effect in patients with high VEGFA & PLGF
Progression Free Survival

placebo    n=66/51, med=198
low dose   n=60/31, med=293
high dose        n=69/38, med=267

**Figure 8:** SEQ ID NO:1, Exemplary amino acid sequence of VEGFA.

```
            10          20          30          40          50          60
MNFLLSWVHW  SLALLLYLHH  AKWSQAAPMA  EGGGQNHHEV  VKFMDVYQRS  YCHPIETLVD

            70          80          90         100         110         120
IFQEYPDEIE  YIFKPSCVPL  MRCGGCCNDE  GLECVPTEES  NITMQIMRIK  PHQGQHIGEM

           130         140         150         160         170         180
SFLQHNKCEC  RPKKDRARQE  KKSVRGKGKG  QKRKRKKSRY  KSWSVYVGAR  CCLMPWSLPG

           190         200         210         220         230
PHPCGPCSER  RKHLFVQDPQ  TCKCSCKNTD  SRCKARQLEL  NERTCRCDKP  RR
```

**Figure 9**: SEQ ID NO:2, Exemplary amino acid sequence of VEGFR2.

```
        10          20          30          40          50          60
MQSKVLLAVA  LWLCVETRAA  SVGLPSVSLD  LPRLSIQKDI  LTIKANTTLQ  ITCRGQRDLD

        70          80          90         100         110         120
WLWPNNQSGS  EQRVEVTECS  DGLFCKTLTI  PKVIGNDTGA  YKCFYRETDL  ASVIYVYVQD

       130         140         150         160         170         180
YRSPFIASVS  DQHGVVYITE  NKNKTVVIPC  LGSISNLNVS  LCARYPEKRF  VPDGNRISWD

       190         200         210         220         230         240
SKKGFTIPSY  MISYAGMVFC  EAKINDESYQ  SIMYIVVVVG  YRIYDVVLSP  SHGIELSVGE

       250         260         270         280         290         300
KLVLNCTART  ELNVGIDFNW  EYPSSKHQHK  KLVNRDLKTQ  SGSEMKKFLS  TLTIDGVTRS

       310         320         330         340         350         360
DQGLYTCAAS  SGLMTKKNST  FVRVHEKPFV  AFGSGMESLV  EATVGERVRI  PAKYLGYPPP

       370         380         390         400         410         420
EIKWYKNGIP  LESNHTIKAG  HVLTIMEVSE  RDTGNYTVIL  TNPISKEKQS  HVVSLVVYVP

       430         440         450         460         470         480
PQIGEKSLIS  PVDSYQYGTT  QTLTCTVYAI  PPPHHIHWYW  QLEEECANEP  SQAVSVTNPY

       490         500         510         520         530         540
PCEEWRSVED  FQGGNKIEVN  KNQFALIEGK  NKTVSTLVIQ  AANVSALYKC  EAVNKVGRGE

       550         560         570         580         590         600
RVISFHVTRG  PEITLQPDMQ  PTEQESVSLW  CTADRSTFEN  LTWYKLGPQP  LPIHVGELPT

       610         620         630         640         650         660
PVCKNLDTLW  KLNATMFSNS  TNDILIMELK  NASLQDQGDY  VCLAQDRKTK  KRHCVVRQLT

       670         680         690         700         710         720
VLERVAPTIT  GNLENQTTSI  GESIEVSCTA  SGNPPPQIMW  FKDNETLVED  SGIVLKDGNR
```

**Figure 9 (continued):** SEQ ID NO:2, Exemplary amino acid sequence of VEGFR2.

NLTIRRVRKE DEGLYTCQAC SVLGCAKVEA FFIIEGAQEK TNLEIIILVG TAVIAMFFWL

LLVIILRTVK RANGGELKTG YLSIVMDPDE LPLDEHCERL PYDASKWEFP RDRLKLGKPL

GRGAFGQVIE ADAFGIDKTA TCRTVAVKML KEGATHSEHR ALMSELKILI HIGHHLNVVN

LLGACTKPGG PLMVIVEFCK FGNLSTYLRS KRNEFVPYKT KGARFRQGKD YVGAIPVDLK

RRLDSITSSQ SSASSGFVEE KSLSDVEEEE APEDLYKDFL TLEHLICYSF QVAKGMEFLA

SRKCIHRDLA ARNILLSEKN VVKICDFGLA RDIYKDPDYV RKGDARLPLK WMAPETIFDR

VYTIQSDVWS FGVLLWEIFS LGASPYPGVK IDEEFCRRLK EGTRMRAPDY TTPEMYQTML

DCWHGEPSQR PTFSELVEHL GNLLQANAQQ DGKDYIVLPI SETLSMEEDS GLSLPTSPVS

CMEEEEVCDP KFHYDNTAGI SQYLQNSKRK SRPVSVKTFE DIPLEEPEVK VIPDDNQTDS

GMVLASEELK TLEDRTKLSP SFGGMVPSKS RESVASEGSN QTSGYQSGYH SDDTDTTVYS

SEEAELLKLI EIGVQTGSTA QILQPDSGTT LSSPPV

56

**Figure 10**: SEQ ID NO:3, Exemplary amino acid sequence of PLGF.

```
           10          20          30          40          50          60
MPVMRLFPCF LQLLAGLALP AVPPQQWALS AGNGSSEVEV VPFQEVWGRS YCRALERLVD


           70          80          90         100         110         120
VVSEYPSEVE HMFSPSCVSL LRCTGCCGDE NLHCVPVETA NVTMQLLKIR SGDRPSYVEL


          130         140         150         160         170         180
TFSQHVRCEC RHSPGRQSPD MPGDFRADAP SFLPPRRSLP MLFRMEWGCA LTGSQSAVWP


          190         200         210         220
SSPVPEEIPR MHPGRNGKKQ QRKPLREKMK PERCGDAVPR R
```

**Figure 11:**

Figure 12:

**Figure 13:**

**Figure 14:**

Europäisches
Patentamt

European
Patent Office

Office européen
des brevets

# EUROPEAN SEARCH REPORT

**Application Number**

EP 16 18 4951

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | S. X. YANG ET AL: "Gene Expression Profile and Angiogenic Markers Correlate with Response to Neoadjuvant Bevacizumab Followed by Bevacizumab plus Chemotherapy in Breast Cancer", CLINICAL CANCER RESEARCH, vol. 14, no. 18, 15 September 2008 (2008-09-15), pages 5893-5899, XP055000181, ISSN: 1078-0432, DOI: 10.1158/1078-0432.CCR-07-4762 * abstract * * page 5894, column 2, paragraph 3-5 * * table 1 * | 1-14 | INV. G01N33/574 |
| A | WO 2009/050014 A1 (SIEMENS HEALTHCARE DIAGNOSTICS [DE]; WIRTZ RALPH [DE]; MILDE-LANGOSCH) 23 April 2009 (2009-04-23) * abstract * * page 14, lines 1-11 * * page 16, line 6 - page 17, line 19 * * page 24, lines 17-34 * * page 28, lines 30-31 * | 1-14 | |
| A | WO 2008/088854 A2 (UNIV SOUTHERN CALIFORNIA [US]; LENZ HEINZ-JOSEF [US]) 24 July 2008 (2008-07-24) * abstract * * table 4 * * page 7, lines 9-22 * | 1-14 | **TECHNICAL FIELDS SEARCHED (IPC)**  G01N |

-/--

The present search report has been drawn up for all claims

| Place of search | Date of completion of the search | Examiner |
|---|---|---|
| Munich | 30 January 2017 | Thumb, Werner |

Europäisches
Patentamt

European
Patent Office

Office européen
des brevets

# EUROPEAN SEARCH REPORT

**Application Number**

EP 16 18 4951

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | D. G. DUDA ET AL: "Plasma Soluble VEGFR-1 Is a Potential Dual Biomarker of Response and Toxicity for Bevacizumab with Chemoradiation in Locally Advanced Rectal Cancer", THE ONCOLOGIST, vol. 15, no. 6, 1 June 2010 (2010-06-01), pages 577-583, XP055010526, ISSN: 1083-7159, DOI: 10.1634/theoncologist.2010-0029 * page 579, column 1, paragraph 2 * | 1-14 | |
| A | WO 2010/075420 A1 (GENENTECH INC [US]; YAN YIBING [US]; FIELDER PAUL J [US]; WU QUN JENNY) 1 July 2010 (2010-07-01) * paragraphs [0007] - [0009], [0011], [0014], [0016], [0017], [0036], [0086] - [0088] * * claim 18; figures 1-3 * | 1-14 | |
| A | MILES DAVID W ET AL: "Phase III study of bevacizumab plus docetaxel compared with placebo plus docetaxel for the first-line treatment of human epidermal growth factor receptor 2-negative metastatic breast cancer", JOURNAL OF CLINICAL ONCOLOGY,, vol. 28, no. 20, 24 May 2010 (2010-05-24), pages 3239-3247, XP002660179, ISSN: 1527-7755 * the whole document * | 1-14 | TECHNICAL FIELDS SEARCHED (IPC) |

-/--

The present search report has been drawn up for all claims

| Place of search | Date of completion of the search | Examiner |
|---|---|---|
| Munich | 30 January 2017 | Thumb, Werner |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 16 18 4951

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | AOYAGI ET AL.: "Association of plasma VEGF-A, soluble VEGFR-1 and VEGFR-2 levels and clinical response and survival in advanced colorectal cancer patients receiving bevacizumab with modified FOLFOX6", ONCOLOGY LETTERS, vol. 1, no. 2, 4 February 2010 (2010-02-04), pages 253-259, XP055340368, GR ISSN: 1792-1074, DOI: 10.3892/ol_00000045 * abstract * ----- | 1-14 | |

TECHNICAL FIELDS
SEARCHED     (IPC)

The present search report has been drawn up for all claims

| Place of search | Date of completion of the search | Examiner |
|---|---|---|
| Munich | 30 January 2017 | Thumb, Werner |

EPO FORM 1503 03.82 (P04C01)

## ANNEX TO THE EUROPEAN SEARCH REPORT
## ON EUROPEAN PATENT APPLICATION NO.

EP 16 18 4951

This annex lists the patent family members relating to the patent documents cited in the above-mentioned European search report.
The members are as contained in the European Patent Office EDP file on
The European Patent Office is in no way liable for these particulars which are merely given for the purpose of information.

30-01-2017

| Patent document cited in search report | | Publication date | Patent family member(s) | | Publication date |
|---|---|---|---|---|---|
| WO 2009050014 | A1 | 23-04-2009 | EP | 2065399 A1 | 03-06-2009 |
| | | | EP | 2201037 A1 | 30-06-2010 |
| | | | EP | 2215116 A1 | 11-08-2010 |
| | | | US | 2011033852 A1 | 10-02-2011 |
| | | | WO | 2009050014 A1 | 23-04-2009 |
| | | | WO | 2009068445 A1 | 04-06-2009 |
| WO 2008088854 | A2 | 24-07-2008 | AU | 2008205543 A1 | 24-07-2008 |
| | | | CA | 2675352 A1 | 24-07-2008 |
| | | | EP | 2109686 A2 | 21-10-2009 |
| | | | US | 2010092485 A1 | 15-04-2010 |
| | | | US | 2012020984 A1 | 26-01-2012 |
| | | | WO | 2008088854 A2 | 24-07-2008 |
| WO 2010075420 | A1 | 01-07-2010 | AU | 2009329994 A1 | 07-07-2011 |
| | | | AU | 2014203615 A1 | 17-07-2014 |
| | | | BR | PI0918211 A2 | 08-12-2015 |
| | | | CA | 2746120 A1 | 01-07-2010 |
| | | | CN | 102265157 A | 30-11-2011 |
| | | | EP | 2382472 A1 | 02-11-2011 |
| | | | ES | 2541925 T3 | 28-07-2015 |
| | | | IL | 213410 A | 26-02-2015 |
| | | | JP | 5416221 B2 | 12-02-2014 |
| | | | JP | 2012513592 A | 14-06-2012 |
| | | | KR | 20110106899 A | 29-09-2011 |
| | | | KR | 20140020368 A | 18-02-2014 |
| | | | KR | 20160021308 A | 24-02-2016 |
| | | | SG | 172355 A1 | 28-07-2011 |
| | | | SG | 187385 A1 | 28-02-2013 |
| | | | SG | 102201604988Y A | 28-07-2016 |
| | | | US | 2010216115 A1 | 26-08-2010 |
| | | | US | 2013022596 A1 | 24-01-2013 |
| | | | US | 2013224193 A1 | 29-08-2013 |
| | | | US | 2013224194 A1 | 29-08-2013 |
| | | | WO | 2010075420 A1 | 01-07-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0939319 A

- EP 1610129 A

**Non-patent literature cited in the description**

- **SALTZ et al.** *J. Clin. Oncol.,* 2008, vol. 26, 2013-2019
- **YANG et al.** *Clin. Cancer Res.,* 2008, vol. 14, 5893-5899
- **HURWITZ et al.** *N. Engl. J. Med.,* 2004, vol. 350, 2335-2342
- **FERRARA.** *Mol. Biol. Cell,* 2010, vol. 21, 687
- **LEUNG et al.** *Science,* 1989, vol. 246, 1306
- **HOUCK et al.** *Mol. Endocrin.,* 1991, vol. 5, 1806
- **KIM et al.** *Growth Factors,* 1992, vol. 7 (1), 53-64
- **THOMPSON.** *Nucl. Acids Res.,* 1994, vol. 2, 4673-4680
- **BRUTLAG.** *Comp. App. Biosci.,* 1990, vol. 6, 237-245
- **ALTSCHUL.** *Nucl. Acids Res.,* 1997, vol. 25, 3389-3402
- **ALTSCHUL.** *J. Mol. Evol.,* 1993, vol. 36, 290-300
- **ALTSCHUL.** *J. Mol. Biol.,* 1990, vol. 215, 403-410
- **HENIKOFF.** *PNAS,* 1989, vol. 89, 10915
- **RUCZINSKI, I. et al.** *J. of Computational and Graphical Statistics,* 2003, vol. 12, 475-511
- **FRIEDMAN, J. H.** *J. of the American Statistical Association,* 1989, vol. 84, 165-175
- **HASTIE ; TREVOR ; TIBSHIRANI ; ROBERT ; FRIEDMAN ; JEROME.** The Elements of Statistical Learning. Springer Series in Statistics, 2001
- **BREIMAN, L. ; FRIEDMAN, J. H. ; OLSHEN, R. A. ; STONE, C. J.** *Classification and regression trees,* 1984

- **BREIMAN, L. ; RANDOM FORESTS.** *Machine Learning,* 2001, vol. 45, 5-32
- **PEPE, M. S.** The Statistical Evaluation of Medical Tests for Classification and Prediction. Oxford Statistical Science Series, 2003, 28
- **DUDA, R. O. ; HART, P. E. ; STORK, D. G.** Pattern Classification. Wiley Interscience, 2001
- **KOHLER et al.** Hybridoma Techniques. Cold Spring Harbor Laboratory, 1980
- **TIJSSEN.** Practice and Theory of Enzyme Immunoassays. Elsevier, 1985
- **CAMPBELL.** Monoclonal Antibody Technology. Elsevier, 1984
- **HURRELL.** Monoclonal Hybridoma Antibodies: Techniques and Applications. CRC Press, 1982
- **ZOLA.** Monoclonal Antibodies: A Manual of Techniques. CRC Press, Inc, 1987, 147-1 58
- **FERRARA, N.** *Mol. Biol. of the Cell,* 2010, vol. 21, 687-690
- **EISENHAUSER et al.** New response evaluation criteria in solid tumours: Revised RECIST guideline (version 1.1). *Eur. J. Cancer,* 2009, vol. 45, 228-247
- **LOTTSPEICH.** Bioanalytik. Spektrum Akademisher Verlag, 1998
- **SAMBROOK ; RUSSELL.** Molecular Cloning: A Laboratory Manual,. CSH Press, 2001
- **MILES.** *J. Clin. Oncol.,* 24 May 2010